# EUROPEAN PATENT APPLICATION

(11) **EP 1 359 154 A1**
(43) Date of publication of application: **05.11.2003**
(21) Application number: 02009663.2
(22) Date of filing: 29.04.2002
(51) Int. Cl.: C07J 53/00

(54) **Further syntheses of cyproterone acetate**

(71) Applicant: Boehringer Ingelheim International GmbH, 55216 Ingelheim (DE)
(72) Inventor: Manosroi, Aranya, Nong Hoi, Muang, Chiang Mai 50000 (TH); Manosroi, Jiradej, Nong Hoi, Muang, Chiang Mai 50000 (TH); Buddhasukh, Duang, Amphur Muang, Chiang Mai 50200 (TH); Sripalakit, Pattana, Amphur Muang, Uttaradit 53000 (TH); Maier, Roland, 88400 Biberach an der Riss (DE); WERNER, Rolf, 88400 Biberach an der Riss 1 (DE)

(57) **Abstract**

The present invention relates to improved methods for synthesising cyproterone acetate (17α-Acetoxy-6-chloro-1α, 2α-methylene-4,6-pregnadiene-3,20-dione) from solasodine. The methods of the invention are shorter as those of the prior art and therefore more economic.

## Description

### Field of the invention

The present invention relates to improved methods for synthesising cyproterone acetate (17α-Acetoxy-6-chloro-1α, 2α-methylene-4,6-pregnadiene-3,20-dione).

### Background Art

Cyproterone acetate (17α-Acetoxy-6-chloro-1α, 2α-methylene-4,6-pregnadiene-3,20-dione, CAS 427-51-0) of formula (M) 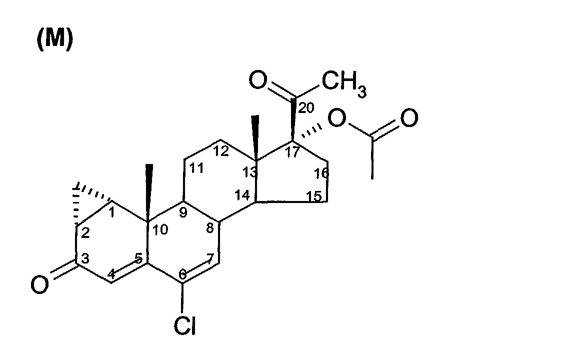 has first been described in DE-AS 11 58 966 as a compound with strong gestagenic activity. Later is was found that it was also a potent androgen antagonist (Neumann. The antiandrogen cyproterone acetate: discovery, chemistry, basic pharmacology, clinical use and tool in basic research. Exp Clin Endocrinol (1994), 102: 1-32). The compound nowadays is a valuable drug present in pharmaceutical compositions for various indications.

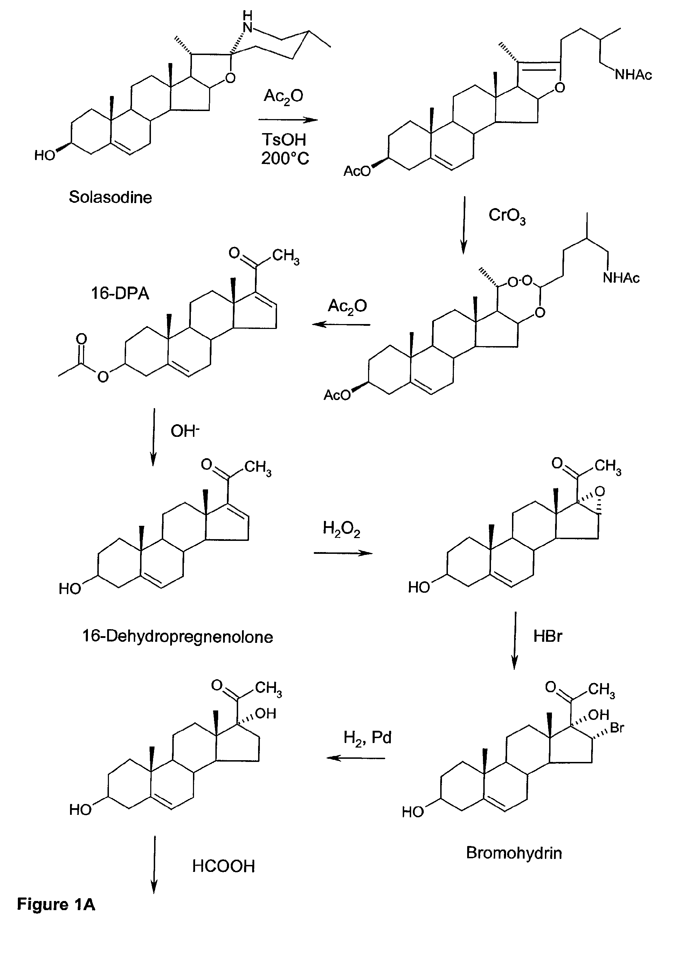

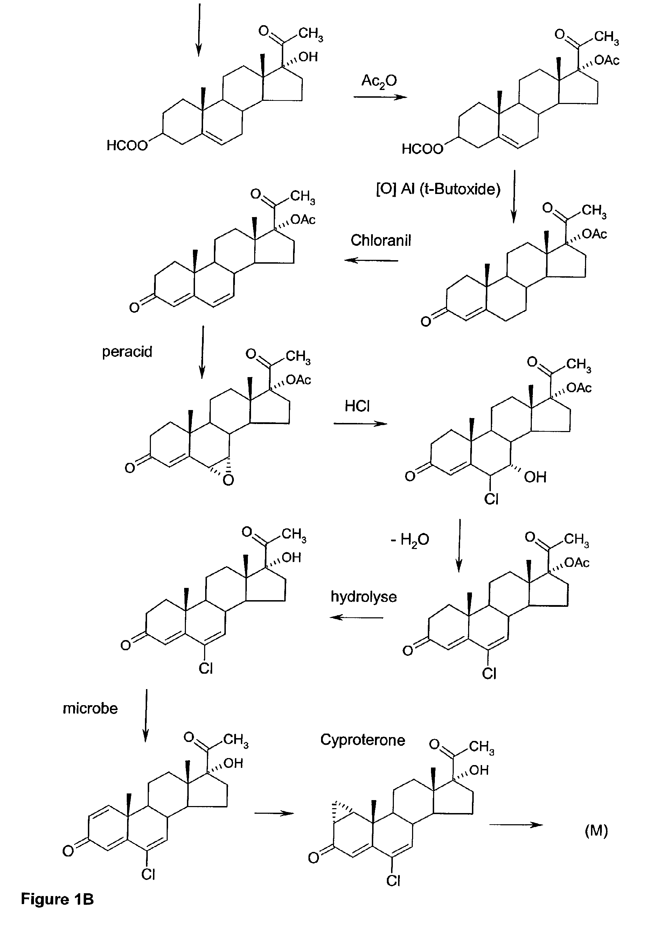

There are various strategies for the chemical synthesis of the compound, see for example DE-OS 40 06 165, or Neumann, *supra.* Conventional synthesis starts from solasodine of formula (A) 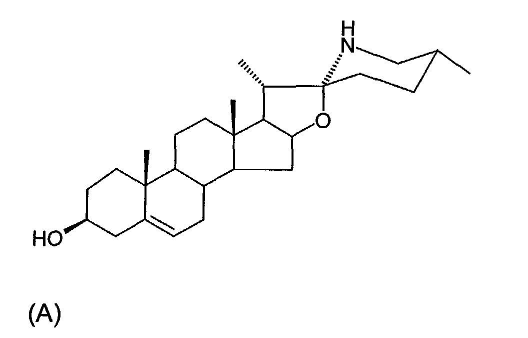 which can be extracted from leaves of the tropic plant *Solanum laciniatum,* Ait, and involves a laborious 18 step procedure acoording to Figure 1 (Sree, Rao and Mahapatra, Research and Industry, Vol. 27, 1982, pp. 326-328; Ringold, et al., J. Am. Chem. Soc., Vol. 78, 1976, pp. 816-819; US 3,485,852; DE 1075114; Ringold et al., J. Am Chem. Soc., 81, 3485 (1959); GB 890315; The Merck Index, 12th Edition, 1996; The Merck Index, 12thEdition, 1996; US 3,234,093).

### Disclosure of the invention

The present invention now provides improved synthetic methods for this valuable compound which are both faster and more cost-effective. In particular, by the present invention it is possible to synthesise cyproterone acetate from solasodine in 14 or 16 steps as opposed to the conventional 18 step synthesis.

To achieve that improvement, the present invention provides some reaction steps which are of particular advantage. Therefore, in one embodiment it relates to a process for the production of cyproterone acetate (M), comprising
(a) Converting 6,7α-oxido-4-pregnene-17α-ol-3,20-dione-17-acetate (J₂) into chlormadinone acetate (K₂) by a single step procedure;
(b) Introducing a double bond at position 1 of (K₂) to yield delmadinone acetate (L₂); and
(c) Introducing a methylene group bridging positions 1 and 2 of (L₂) to yield cyproterone acetate (M).

The following scheme illustrates that process:

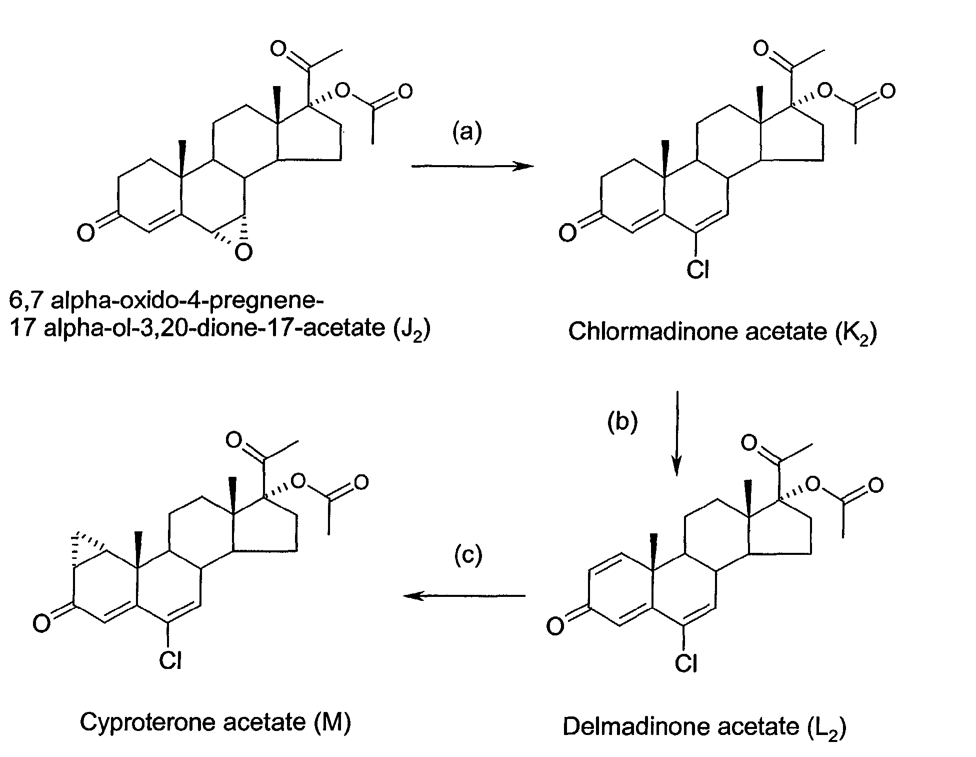

Preferably, step (a) is carried out by passing anhydrous hydrogen chloride gas through a reaction mixture containing 6,7α-oxido-4-pregnene-17α-ol-3,20-dione-17-acetate (J₂). Alternatively, using hydrochloric acid in aqueous dioxane yields the intermediate chlorohydrin which can be dehydrated to chlormadinone acetate (Bruckner, Hampel, and Johnson, Chem. Ber., 94,1225, 1961; Lednicer and Mitscher, The Organic Chemistry of Drug Synthesis, John Wiley and Sons, New York, 1977).

In a further preferred embodiment, step (b) is carried out by the use the dehydrogenation agent 2,3-dichloro-5,6-dicyano-1,4-benzoquinone (DDQ, CAS 84-58-2). Dioxane may be used as a sovent. Alternatively, selenium dioxide may be used, e.g. with pyridine and t-butanol as solvent (US Patent 3485852), or according to the method of Ringold et al., J. Amer. Soc., 81, 1959, 3485). A further way to carry out that step is with Pb(OAc )₄ in acetic acid (Abe, T ., Amer. Chem.

Pharm, Bull., 21 (6), 1973, 1295-1299). Furthermore, DDQ may be used together with 4-nitro-phenol in benzene (Abe T ., Kambegawa, A., Chem. Pharm. Bull., 22, 1974, 2824-2829).

Advantageously, step (c) is carried out using the C₁ reagent precursor trimethyl sulfoxonium iodide (TMSI, CAS 1774-47-6) and an alkali hydride. DMSO may be used as a solvent (Goto, G. et al., Chem. Pharm. Bull., 26,1978, 1718-1728). Alternatively, this step may be achieved with diazomethane and treatment with acid (Krakower and Van Dine, J. Org. Chem, 31, 3467 (1966). A further way would be employing diazomethane and hydrolysis according to Wiechert and Kaspar, Chem. Ber., 93,1710 (1960). Another reaction pathway would be use of diazomethane and pyrolysis by loss of nitrogen (Lednicer D and Mitscher LA, The organic chemistry of drug synthesis, Volume 2, A Wiley-Interscience Publication, 1980, pp. 166). Multistep embodiments are described by Tolf et al., Tetrahedron Lett, 25,43, 1984, 4855- 4858.).

Acoordingly, in another aspect the present invention relates to a process for the production of cyproterone actetate (M), comprising reacting delmadinone acetate (L₂) with trimethyl sulfoxonium iodide (TMSI) and an alkali hydride, preferably sodium hydride. The reaction may be carried out in dimethylsulfoxide (DMSO).

In a preferred embodiment, 6,7α-oxido-4-pregnene-17α-ol-3,20-dione-17-acetate (J₂) is obtained by a process comprising
(d) Introducing a double bond at position 6 of 17α-acetoxyprogesterone (H) to convert it into 4,6-pregnadiene-17α-ol-3,20-dione-17-acetate (I₂);
(e) Converting the double bond at position 6 of (I₂) into an epoxy function to yield 6,7α-oxido-4-pregnene-17α-ol-3,20-dione-17-acetate (J₂).

That process is shown in the following scheme:

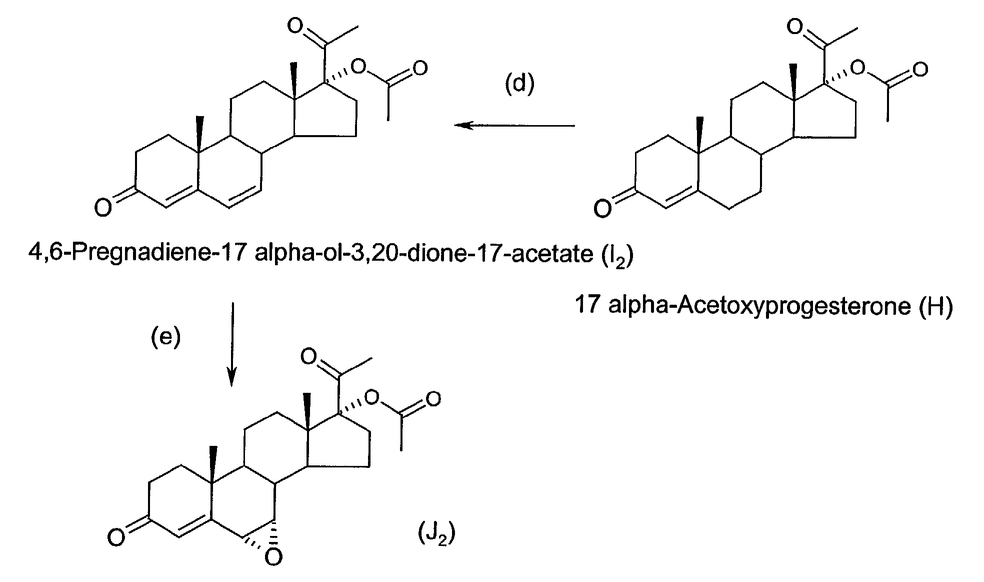

Step (d) may advantageously be performed with chloranil (tetrachloro-p-benzoquinone, CAS 118-75-2), either alone or in combination with selenium dioxide (Lednicer D, Mitscher LA, The organic chemistry of drug synthesis, Vol. 2, A Wiley-lnterscience Publication, 1980, pp. 182.). In another embodiment, chloranil in t-butanol or xylene may be used (L.F. Fieser and M. Fieser, Reagents for organic synthesis, Vol. 1, John Wiley and Sons, Inc., New York, 1967). A further alternative is DDQ and TsOH as catalyst (L.F. Fieser and M. Fieser, Reagents for Organic synthesis, Vol. 2, John Wiley and Sons, Inc., New York,1969.)

Step (e) can be done with m-chloro-perbenzoic acid or monoperoxyphthalic acid. Alternatively, perbenzoic acid with ethylene chloride as solvent could be used (US Patent 3,234,093), or perbenzoic acid according to the method of Lednicer and Mitscher, The Organic chemistry of drug synthesis, Vol.2, A Wiley-Interscience Publication, 1980, pp. 166.

The 17α-acetoxyprogesterone (H) can be prepared conveniently from solasodine by methods known in the art. Solasodine may be obtained e.g. by alcoholic extraction of dried leaves or fresh or dried green berries of *Solanum laciniatum,*

Ait, using e.g. 2-propanol or methanol as extraction agent, and subsequent hydrolysis of the thus obtained glycoside solasonine. The primary extraction product solasonine may be precipitated from the extract e.g. by adding hot water and ammonia solution to the hot extract and allowing to cool. Usually, the crude precipitate is further purified by washing and recrystallization steps, as exemplified in example 1, before acid hydrolysis e.g. with hydrochloric acid, which gives solasodine hydrochloride. Free solasodine may then be obtained by recrystallization from a basic alcoholic solution. Solasodine may then be converted to 16-dehydropregnenolone acetate (16-DPA) by refluxing it in acetic acid/acetic anhydride e.g. in the presence of catalytic p-toluene sulfonic acid (TsOH) and/or pyridine, then oxidising e.g. with chromic anhydride, chromium trioxide, or sodium dichromate, and refluxing again with acetic anhydride.

In one aspect, the present invention relates to a process for the production of 16-dehydropregnenolone actetate (B), comprising
(k) Acetylating solasodine (A) to give *O, N*-diacetylsolasodine;
(I) Isomerising *O, N*-diacetylsolasodine to give pseudosolasodine diacetate;
(m)Oxidising the resulting pseudosolasodine diacetate in the presence of a phase transfer catalyst.

As outlined above, acetylation of solasodine may be achieved reacting it with acetic anhydride in the presence of a base and/or p-toluenesulfonic acid in catalytic amounts. The base may be pyridine. Isomerisation may be obtained with acetic acid. Preferably, the phase transfer catalyst in the oxidation step is tetraethylammonium iodide or tetrabutylammonium hydrogen sulfate, preferably the latter. As oxidising agent, potassium dichromate, sodium dichromate, or potassium permanganate may be used, potassium dichromate being preferred. Methylene chloride may be used as a solvent. Oxidation is carried out in at an acid pH which may be achieved e.g. with sulfuric acid. Hydrolysis of the oxidative product produces 16-DPA.

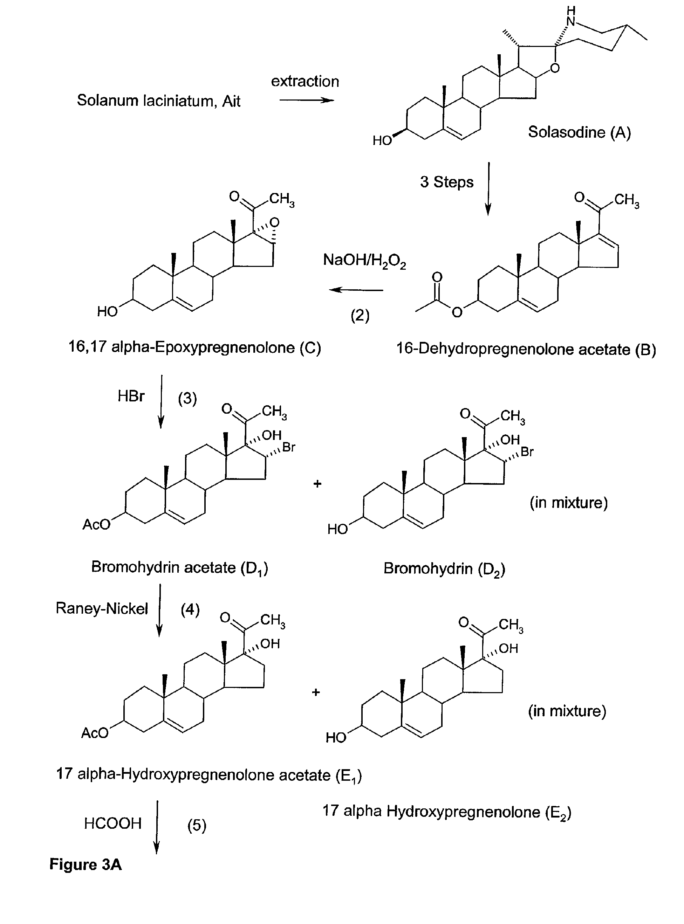

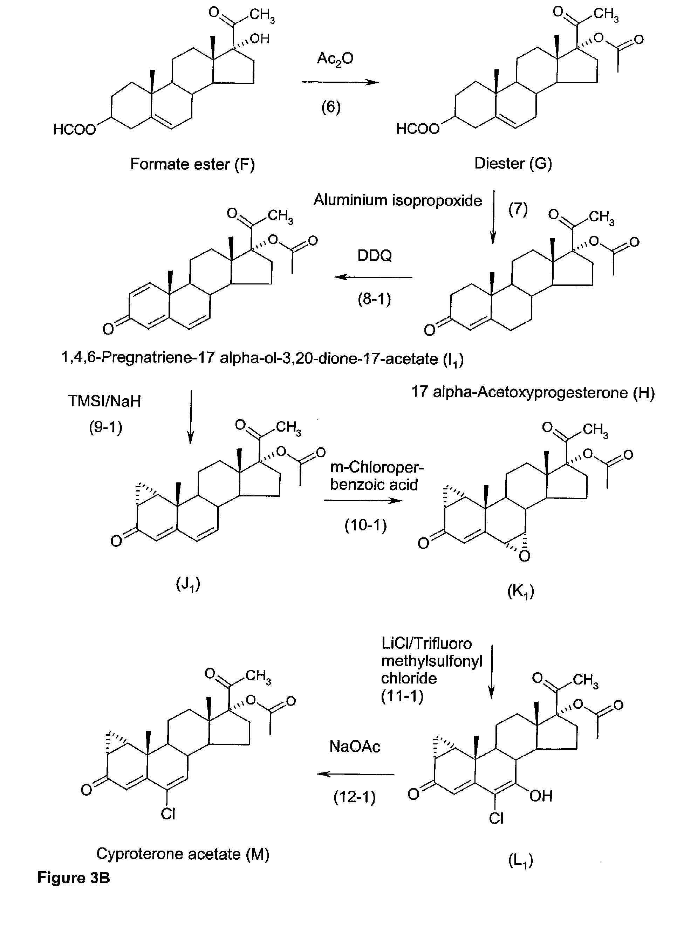

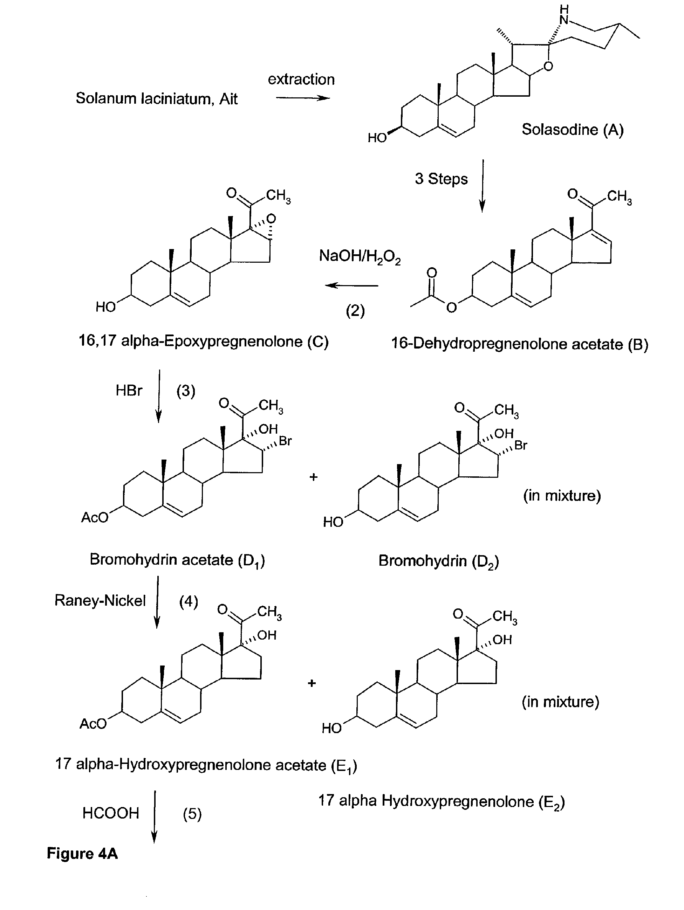

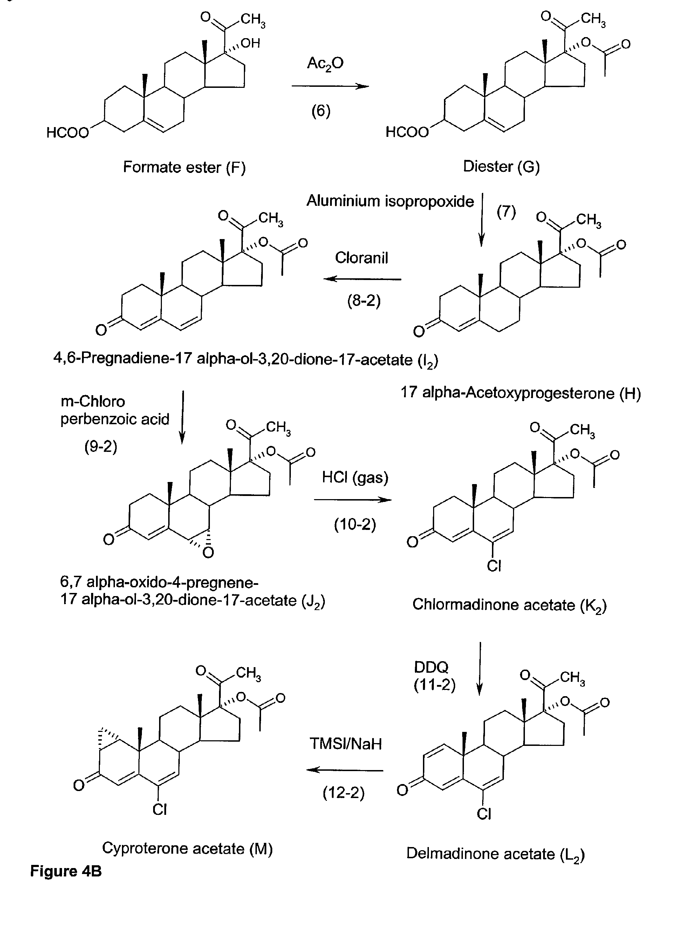

It is of particular advantage to convert the 16-DPA (B) to 16,17α-epoxypregnenolone (C) in a single-step procedure. This may be achieved by reacting (B) with peroxide, preferably hydrogen peroxide, in a basic solution, preferably an alkaline alcoholic solution. Methanol may be employed as a solvent in this reaction, and sodium hydroxide as a base. 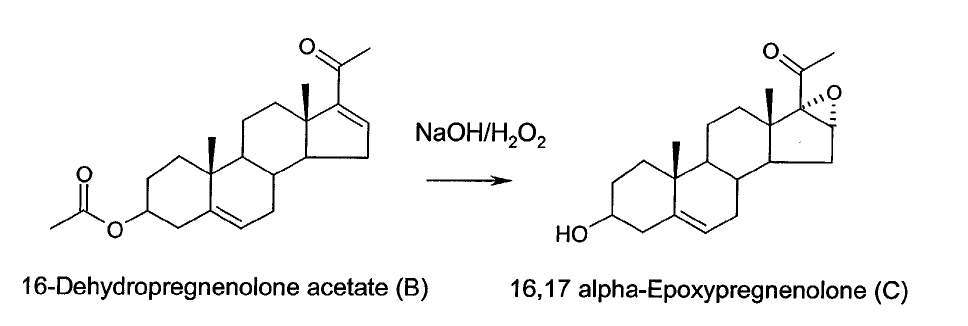 (C) may then be converted to 16-bromo-17α-hydroxy-pregnenolone e.g. by addition of HBr, e.g. in glacial acectic acid, which gives a mixture of the bromohydrin (D₁) and the bromohydrin actetate (D₂) (cf. Figures 3A, 4A). The bromohydrin may then be reduced to 17α-hydroxypregnenolone (E₂, in mixture with the acetate E₁, Δ⁵-pregnene-3β, 17α-diol-20-one-3-acetate) using a catalyst like Raney-Nickel and, converted to the 3-formate ester (F) by reaction with formic acid. This can be then converted to the 17-acetate diester (G) by reaction with acetic anhydride and catalytic acid, e.g. p-toluene sulfonic acid. (G) may be reacted with Aluminium propoxide to yield 17α-acetoxy-progesterone (H) (see Figures 3A, 4A).

In a further embodiment, the present invention provides a process for the production of cyproterone acetate (M), comprising
(f) Introducing two double bonds at positions 1 and 6 of 17α-acetoxyprogesterone (H) to convert it into the 1,4,6-triene compound (I₁) by a single step procedure;
(g) Introducing an methylene group bridging positions 1 and 2 to yield the 1,2α-methylene compound (J₁);
(h) Introducing an oxido group bridging positions 6 and 7 of (J₁) to yield the 6,7α-epoxy compound (K₁);
(i) Transforming the epoxy group of (K₁) to the 6-chloro-7-hydroxy compound (L₁); and
(j) Converting (L₁) to cyproterone acetate (M).

This reaction sequence is illustrated by the following scheme:

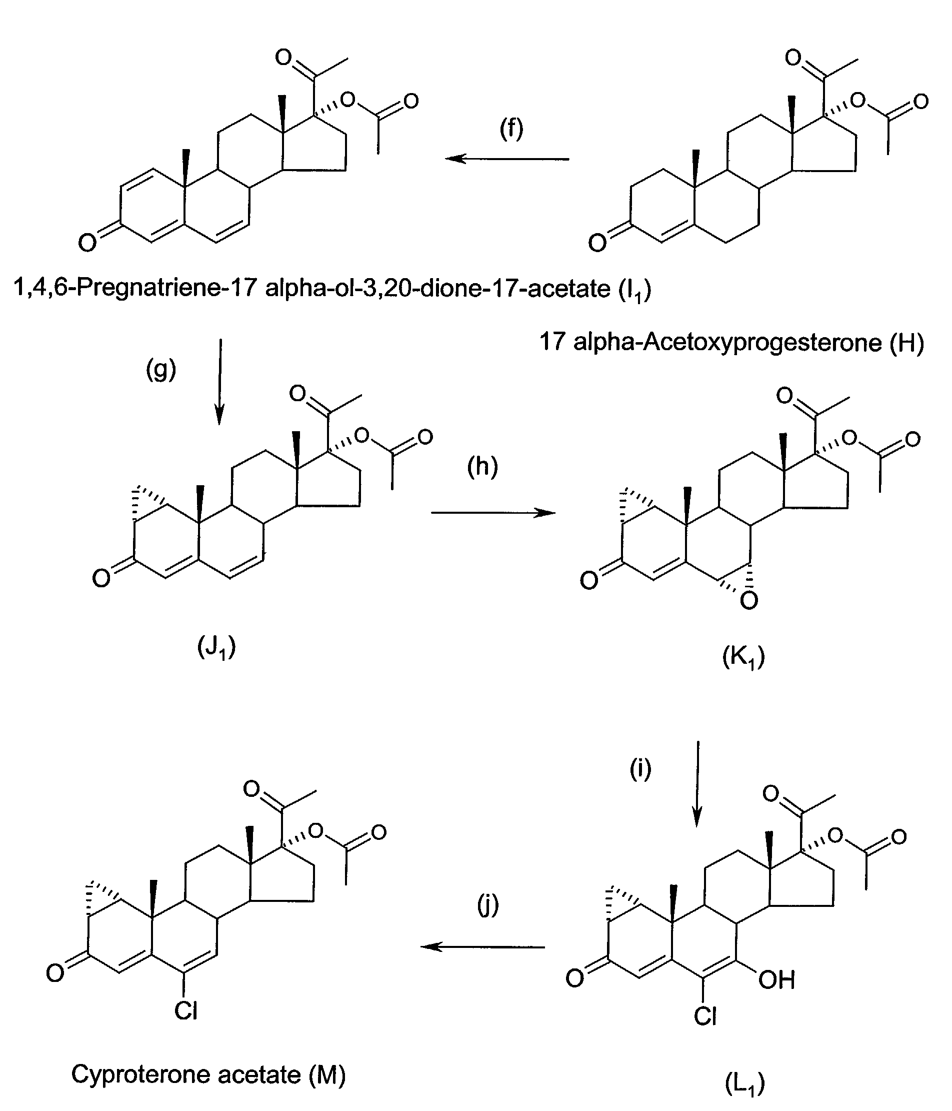

A preferred agent for step (f) is the dehydrogenation agent 2,3-dichloro-5,6-dicyano-1,4-benzoquinone (DDQ, CAS 84-58-2; Muller, M., et al., Helvetica Chemica Acta, 63, 1878, 1980). Alternatively, bromine in absolute dioxane gives dibromide (intermediate) which is reacted with collidine (US 2,962,510).

Advantageously, step (g) is carried out using the C₁ reagent precursor trimethyl sulfoxonium iodide (TMSI, CAS 1774-47-6) and an alkali hydride. Alternatively, this step may be achieved with diazomethane and treatment with acid (Krakower and Van Dine, J. Org. Chem, 31, 3467 (1966). A further way would be employing diazomethane and hydrolysis according to Wiechert and Kaspar, Chem. Ber., 93,1710 (1960). Another agent would be diazomethane and pyrolysis by loss of nitrogen (Lednicer D and Mitscher LA, The organic chemistry of drug synthesis, Volume 2, A Wiley-Interscience Publication, 1980, pp. 166). Multistep embodiments are described by Tolf et al., Tetrahedron Lett, 25,43, 1984, 4855-4858).

Step (h) may be carried out using m-chloroperbenzoic acid (CAS 937-14-4). In one embodiment, ethylene chloride is used as solvent (US 3,234,093). Alternatively, epoxidation of C-6 double bond with m-chloroperoxybenzoic acid (m-CPBA) can be achieved according to Shibata et al., Chem. Pharm. Bull, 40 (4), 935-941 (1992).

For step (i), trifluoromethanesulfonyl choride (CAS 421-83-0) is an agent of choice, preferably in combination with lithium chloride. Alternatively, N,N-dimethyl acetamide hydrochloride in DMSO at 75°C (Uthai Sakee, Boonsong Kongkathip and Nganpong Kongkathip, Abstract of the 27^{th} Science and Technology Conference of Thailand, p. 234) can be used. Finally, step (j) can be achieved employing an alkali acetate like sodium acetate.

Accordingly, in another aspect, the present invention relates to a process for the production of cyproterone actetate (M), comprising reacting compound (K₁) of formula 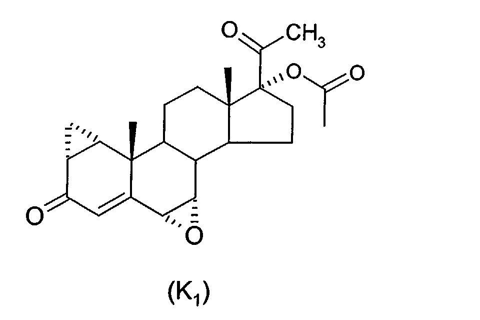 with lithium chloride/trifluoromethylsulfonylchloride to yield compound (L₁) of formula 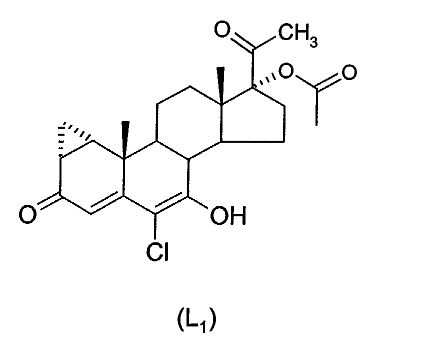 and reacting compound (L₁) with an aqueous acetate solution, preferably a solution of sodium acetate, to yield cyproterone acetate (M).

Again, the 17α-acetoxyprogesterone (H) can be prepared conveniently from solasodine by methods known in the art as described above.

In another aspect, the present invention relates to a process of production of delmadinone acetate (L₂) comprising adding chlormadinone acetate (K₂) to a culture of a microorganism capable of converting (K₂) into (L₂), and isolating the resulting delmadinone actetate from the culture. The organism is preferably *Arthrobacter simplex* or *Bacillus sphaericus*, more preferably the strains ATCC 6946 or ATCC 13805 which may be obtained from the American Type Culture Collection (ATCC), P.O.Box 1549, Manassas, VA 20108, USA. Preferably, an exogenous electron carrier is added to the culture, e.g. menadione (2-Methyl-1,4-naphthoquinone) at a concentration of 0.1 to 1.0 mmol/l, preferably 0.2 to 0.4 mmol/l. Furthermore, the yield may be improved by addition of a steroid to the culture, preferably hydrocortisone at a concentration of 0.01 to 0.55 mmol/l, more preferably 0.3 to 0.5 mmol/l. Good results were obtained when the media contained 5 % dimethylformamide (DMF) to improve the solubility of the steroid. The yield may be further improved by addition of a surfactant, preferably a nonionic detergent, more preferably polyoxyethylenesorbitan monooleate (Tween 80™) at a concentration of 0.25 to 1.0 % (w/v), more preferably 0.5 to 1.0 %. In *B. sphaericus*, 0.1 to 0.2 mmol/l chlormadinone acetate as a starting concentration were found to be optimal, in *A. simplex* good starting concentrations were 0.2 to 0.3 mmol/l chlormadinone actetate. Although the overall yield of the process is lower than with chemical synthesis, it is of advantage particularly in large-scale production because it affords to avoid large amounts of toxic reagent (DDQ) and organic solvent (dioxane).

In the context of this invention, the term "reaction mixture" comprises both homogeneous solutions as well as heterogenous mixtures composed of liquid and/or solid components, like suspensions, slurries, and the like.

### Figure Legends

Figure 1 outlines the conventional 18 step synthesis of cyproterone acetate from solasodine.

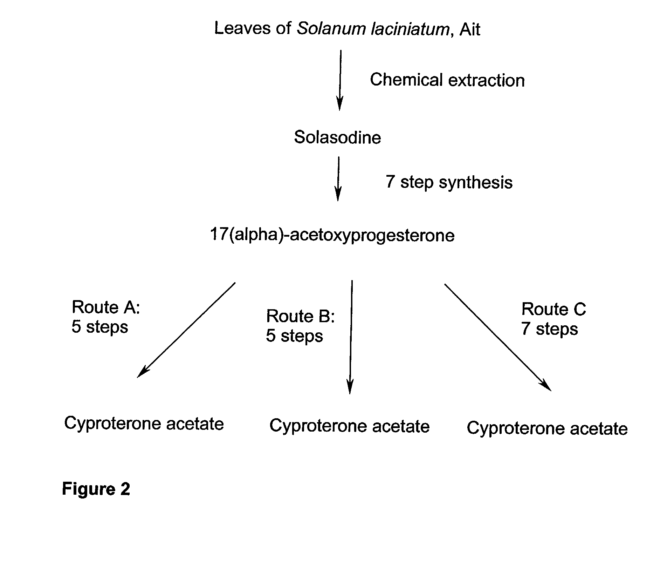

Figure 2 gives an overview of the experimental outline of the synthesis routes according to the present invention.
Figure 3 outlines cyproterone acetate synthesis from solasodine according to Route A of the present invention.
Figure 4 outlines cyproterone acetate synthesis from solasodine according to Route B of the present invention.

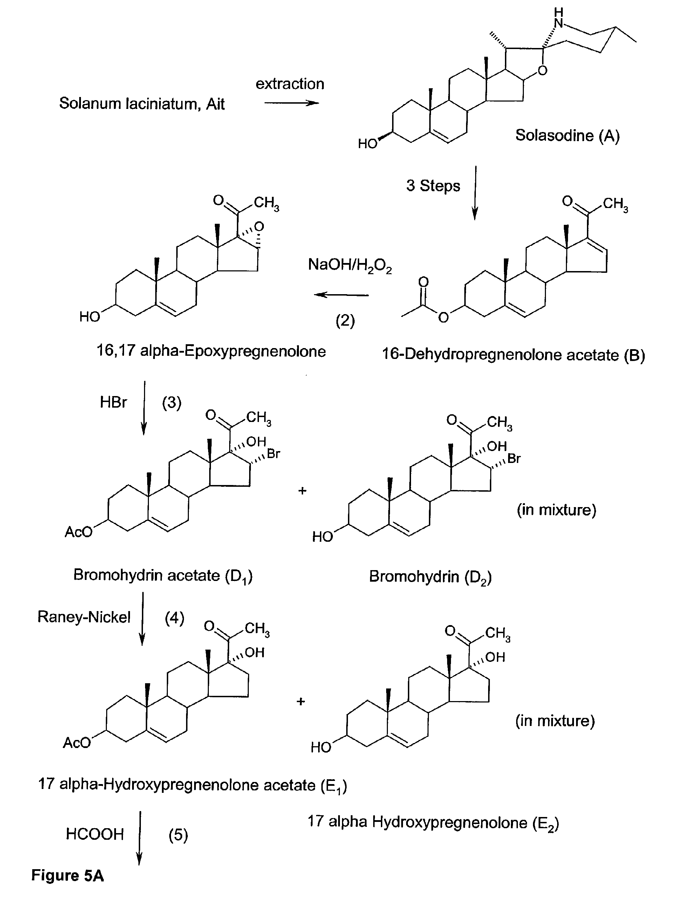

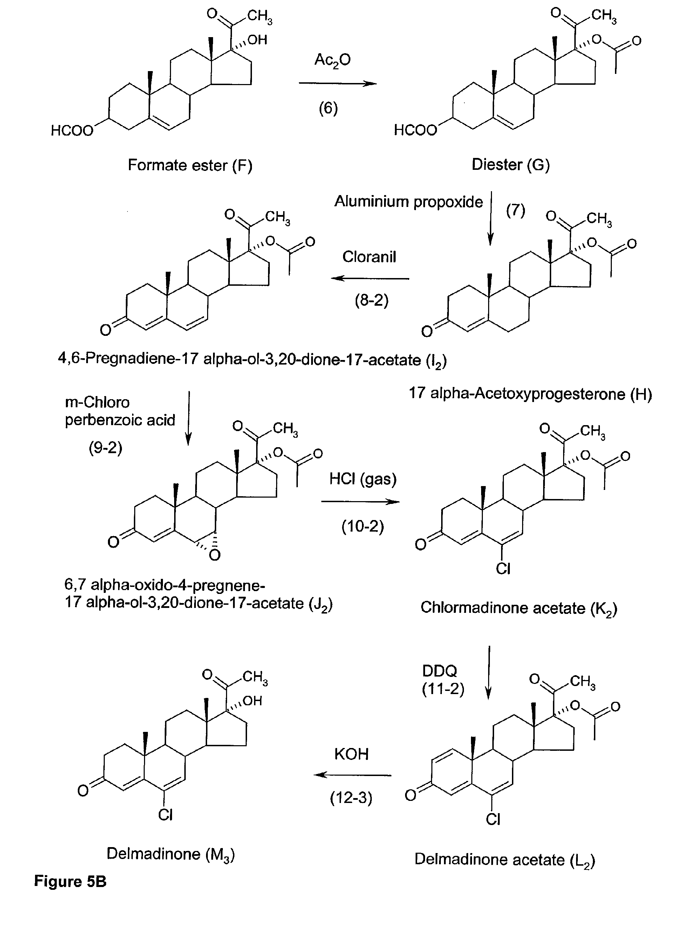

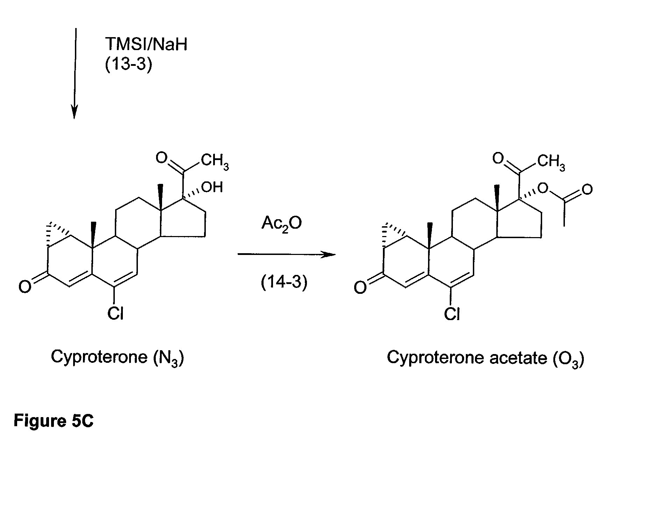

Figure 5 outlines cyproterone acetate synthesis from solasodine according to Route C of the present invention.

### Example 1: Extraction of Solasodine from Leaves of Solanum laciniatum, Ait

### 1. PCRNC Method

### 1.1 Isolation of Solasonine

500 g of ground dried leaves are placed in a two-litre Erlenmeyer flask, 1400 ml of aqueous isopropanol (80 % v/v) are added and the mixture is refluxed with boiling for 4 hours. The extraction mixture is filtered and the residue is re-extracted for 5 times. All six filtrates are pooled. The combined filtrates are concentrated by a rotary evaporator until all isopropanol in the filtrates is evaporated. The mixture is allowed to stand overnight and then filtered. The filtrate is heated to 80 °C and an equal volume of water is added. The mixture is then allowed to stand for one hour and agitated magnetically. 25 % aqueous ammonia is added until the precipitate is formed. Agitation at 80 °C is continued for one hour. The mixture is transferred to a 2-litre separation funnel and allowed to stand over night. The supernatant liquid is then decanted off and the precipitate is exhaustively washed with water until the supernatant is colourless. The decolorization may also be done by electrolysis or bleaching with H₂O₂. The mixture is filtered through filter paper and the filtrate is discarded. The precipitate of glycoalkaloids is dried at 60 °C. Solasodine and solamargine were isolated from the glycoalkaloids by column chromatography.

### 1.2 Hydrolysis of Soiasonine/soiamargine

10 g of crude solasonine glycoside are placed in a 250 -ml round-bottom flask. 100 ml of isopropanol and 8.3 ml of hydrochloric acid (37 % HCI) are added and refluxed in a water bath for 3 hours. Then, it is allowed to cool to room temperature and stand overnight. The mixture is filtered and the precipitate is washed with isopropanol (20 ml) and recrystallised from isopropanol and aqueous sodium hydroxide (50 %). The product (solasodine) is colourless and forms hexagonal plates.
* % yield of solasodine = 0.8 % of the dried leaves (starting from 50 g of the dried leaves).
** The hydrolysis is also done by enzymatic reaction with pure enzyme (galactosidase/glucosidase) or from microorganism *(A. niger, E. coli*, Yeast)

### 2. Alternative methods to extract solasodine from Solanum laciniatum, Ait

### 2.1 Alternative method 1 (modified from R. Culford Bell and Linsay H. Briggs, Journal of the Chemical Society, 1942, pp.1-2)

*Isolation* of *Solasonine.* The dried green berries from shrubs growing in Chiang Mai, Thailand were exhaustively extracted with alcohol. When most of the alcohol was removed from the extract by distillation, an excess of 2 % aqueous acetic acid was added, and the residual alcohol removed by steam-distillation. The aqueous solution was filtered, the filtrate boiled, and the crude solasonine precipitated in a granular form by the addition of ammonia (precipitation in the cold produces a jelly). The crude alkaloid was purified by solution in dilute acetic acid and reprecipitation with ammonia, followed by repeated crystallisation from 60-80 % alcohol and 80 % dioxan-water (crystallisation from higher concentrations of alcohol or dioxan produces a jelly) to give colourless pointed plates, m. p. 284-285 °C (decomp.).

*Hydrolysis* of *Solasonine.* The pure alkaloid was heated with an excess of 3 % hydrochloric acid at 100°C for 3 hours (solasodine hydrochloride, in contrast to solasonine hydrochloride, is only slightly soluble in the cold and is precipitated in a crystalline condition even from the hot solution.). After cooling, the hydrochloride was collected, recystallised twice from 80% alcohol, suspended in hot water, basified with ammonia, and heated at 100°C for ½ hour. The solasodine was collected after cooling and repeatedly crystallised from 80 % alcohol, forming hexagonal plates, m. p. 197.5-198.5°C.

### 2.2 Alternative method 2 (from Linsay H. Briggs and R.C. Cambie, Journal of the Chemical Society, 1958, pp. 1422-5.)

*S.laciniatum,* Ait - *Isolation* of *solasonine.* Fresh green berries (466 g) were coarsely minced and immediately extracted under reflux with boiling methanol for 2 hr. The pulp was separated from the mixture, and washed well with hot methanol, and the filtrate was concentrated under reduced pressure to a small volume under an antifoaming device. After addition of an equal volume of water, the ammonia was added into the boiling solution, and the coagulated precipitate was collected after cooling. The grey glycosidic alkaloid was reprecipitated twice, in a crystalline state, from 3 % acetic acid solution with ammonia. Repeated crystallisation (charcoal) from aqueous methanol (75 %) gave colourless, flat needles (4.10 g) of solasonine, m. p. 301-302 °C (decomp.), with sintering at 296 °C. Chromatography of the partially purified material (m. p. 297 -301 °C) in water-saturated butan-1-ol on alumina (previously stirred and kept for 1 hr with moist butan-1-ol), and developed with butan-1-ol/methanol (1:1), gave a single product (needles from 75% methanol), m. p. 301-302 °C (decomp.), identical with that purified by crystallization alone.

*Hydrolysis of Solasonine - Solanine* (500 mg) in ethanol (10 ml) was hydrolysed by refluxing in concentrated hydrochloric acid (2 ml) for 3 hr. The crystalline hydrochloride (slender needles) deposited after hydrolysis for 30 min was collected after cooling overnight and was washed with water (10 ml). Treatment of a suspension of the hydrochloride with concentrated ammonia (25 ml) for 1 hr at 100 °C gave the crude aglycone. Three crystallisations from methanol yielded large colourless hexagonal plates of solasodine, m. p. and mixed m. p. 196-198 °C. The infrared spectrum was identical with that of authentic solasodine.

### 2.3 Alternative method 3 ( from U.S. Patent 3,960,839, Milton Gverrero, Equador, 1976)

A. The fruit of huapag is picked in the pinton stage. One kg of fruit is used. Each fruit is divided into quarters by two mutually perpendicular cuts along the axis of the fruit. The fruit is then dried in an oven at 60 °C until brittle enough to grind easily. The fruit is then ground in a simple grooved-disc mill set so as to barely avoid fracturing the seeds. The weight of the ground fruit is 270 g. The solids content of the ground fruit is 94 % based on weight loss at 100 °C.
   For the extraction, the ground fruit is placed in a two litre Erlenmeyer flask and 810 ml of aqueous isopropanol (77 % isopropanol by volume) is added. The flask is stoppered and the extraction is carried out at room temperature for 2 hours with alternating 5 minute periods of vigorous manual agitation and rest. The extraction mixture is drained on filter paper in a conical funnel and the solids are returned to the Erlenmeyer flask. For a second extraction, a further portion (540 ml) of aqueous isopropanol is added and the alternate agitation and rest is repeated except that the period is one hour instead of two. The liquids are again drained as before. Then, a third extraction, identical to the second, is carried out. All three filtrates are combined.
   The combined liquids are fed slowly into a one-litre rotary evaporator heated with a water bath at 60°-70°C. The initial pressure is about 150 mm of mercury, and this is gradually reduced to 50 mm by which time the distillate is essentially pure water. The aqueous residue is not allowed to cool, but its weight is adjusted to 510 g by the addition of water heated to 60-70°C. The addition of the water is made over a 2 minute periods, while the mixture is being stirred. A precipitate forms immediately. The mixture is then allowed to stand for 4 hours, during which time it cools to approximately room temperature.
   The supernatant liquid is then decanted through medium filter paper and finally the precipitate (P) is transferred to the filter paper. P is a finely divided but easily filtered material. Its color is olive-drab. After drying at 100 °C, P weight is 1.9 g. This material begins to melt at about 160 °C, but is not yet completely melted at 230 °C.
   The filtrate is placed in a one litre round-bottom flask fitted with a reflux condenser and is then heated to 80°C. It is maintained at this temperature and agitated magnetically while 15 ml of 25 % aqueous ammonia is added, and agitation at 80°C is continued for one more hour. The mixture is immediately filtered through medium paper and the filtrate is discarded. It has a pH between 9 and 10 and is free of glycosides.
   The filter cake, still on the filter paper, is pressed between absorbent papers to remove as much of the liquid as possible. Then, it is dried at 60°C to a final weight of 25.7 g, further drying at 100°C gives a weight loss of 27.7 %. Analysis of a sample of the 25.7 g cake shows that it contains 16.7 g of glycosides and indicates a negligible loss of glycosides in the purification and isolation.
B. In a one-litre round-bottom flask are placed 46 g of damp crude glycoside (prepared as described in part A), 138 g isopropanol, 37 g hydrochloric acid (37.6 % HCI), and 9 g tap water. The flask is equipped with a reflux condenser and magnetic agitation and is heated to reflux in a water bath for 3 hours. At the end of this time the content is a light brown suspension, which is allowed to cool to room temperature over a period of 2 hours. Then, 40 % (weight) sodium hydroxide in water are added. The sodium hydroxide is added in 5 ml increments at first, and later dropwise. Between additions, the mixture is swirled vigorously by hand for one to two minutes. At about pH 9 the viscosity of the mixture decreases noticeably, the colour deepens and the formation of a second liquid phase becomes apparent. Addition of alkali is stopped at an pH of about 9.5 to 10, by which time a considerable separation of the liquid into two phases and the formation of a precipitate is observable. The total amount of sodium hydroxide solution added is 29 ml.

The mixture is allowed to stand for 1½ hr at room temperature and then is filtered by gravity through medium paper. The precipitate is dried by pressing between absorbent papers.

The filtrate consists of two layers. The upper layer is brown and appears to contain the major portion of the isopropanol as well as a very small amount of solasodine, in addition to impurities. The lower layer is dark brown and appears to contain water, sodium chloride and impurities. The entire filtrate is discarded. The damp participate is agitated with aqueous hydrochloric acid is added until the pH is between 6 and 7. It is then allowed to stand overnight. Then, the mixture is filtered by gravity through medium paper and the precipitate is pressed between absorbent papers. The precipitate is dried in an oven at 60°C for 12 hours.

The dry weight of the precipitate is 14 g.

The precipitate is estimated to contain 97 % solasodine on the following basis: Pure solasodine is reported to melt at 200-202 °C. An early, less pure sample produced here melted 191-197 °C and contained 95% solasodine.

### 2.4. Alternative method 4

2.4.1 Materials: Fruits (code No. 160695-3P) and leaves (code No.G1090395) of S. *laciniatum* at the age of 3.5 months grown in Bann Huay Sai District, Chiang Mai, Thailand were collected, dried in an oven at 60°C and ground into powder by a simple grooved-disc mill. The reference standards of solasodine, 16-DPA, and other chemicals were purchased from Sigma Co. (St. Louis, MO, USA). All solvents for chromatographic purposes were of HPLC grade. Other solvents were reagent grade.
2.4.2 Isolation of solasodine from *Solanum laciniatum* Ait.
2.4.2.1 Effects of 2-propanol concentrations on crude glycoside extraction

The dried leaf powder (500 g) was extracted 4-5 times by 0 to 100% v/v of 2-propanol/water (1.5 I each) at 70°C in an Erlenmeyer flask. All filtrates were pooled and 2-propanol was vacuum evaporated using the rotary evaporator (Buchi, Switzerland). The filtrate was heated to 80°C and stirred while adding 30% aqueous ammonia solution until the pH reached 9-10. The formed precipitate was collected, and then crude glycosides were hydrolyzed by 1 N hydrochloric acid in 2-propanol in a round-bottom flask for 3 h. The 20% sodium hydroxide solution was added to precipitate the crude solasodine. The pure solasodine was obtained by crystallization in methanol. Yields were best with 70-80 % (v/v) 2-propanol, with 0.45 % w/w of dry leaves within that range. Particularly suitable for that method is 2-propanol at a concentration of 70 % since it was the lowest concentration giving that high yield of solasodine.

### 2.4.2.2 Comparison of various methods for hydrolysis of glycosides

The dried leaf powder (1 kg) was extracted by the same procedure as in 2.4.2.1 but using distilled water instead of 2-propanol. The crude glycosides were divided into four portions and hydrolyzed by four methods which were electrolysis, 1 N hydrochloric acid in water, ethanol or 2-propanol.

For the electrolysis method, the aqueous extract (1.5 I) in 0.02 N hydrochloric acid was applied with a DC current (30 A) for 2 h using 2 pairs of aluminum plate electrodes (10 x 10 cm²) in a 3 litre tank.

Using 2-propanol as a solvent in the hydrochloric acid hydrolysis gave the highest yield of solasodine.

### 2.4.2.3 Comparison of solasodine contents in fruits and leaves of Solanum laciniatum Ait.

The dried fruit (60-750 g) or leaf (40-750 g) powder was dispersed in 70% 2-propanol and sonicated for 2 h before extraction. The suspension was put into the thimble and the alcoholic solution (volume adjusted to 300 or 3000 ml) was added to the Soxhlet receiving flask. The solution was refluxed until exhaustion and 2-propanol was vacuum evaporated using the rotary evaporator. Boiling water was added to the residue while being stirred. The solution was filtered and the filtrate was heated to 80°C and stirred while adding 30% aqueous ammonia solution until the pH reached 9-10. The precipitate was repeatedly washed with distilled water, filtered and dried at 60°C.

The above crude glycosides, 2-propanol and 37.6% hydrochloric acid in the ratio of 16.4:76:7.6% by weight were placed in a round-bottom flask. The solution was refluxed for 3 h and was then filtered by vacuum pump. The collected precipitate was dissolved in 2-propanol and 20% w/v sodium hydroxide solution. A large amount of water was added into the solution to obtain the crystalline solasodine. Solasodine was filtered through the filter paper and dried at 60°C. Recrystallisation of solasodine from methanol gave colourless hexagonal plates of crystalline solasodine which was used as a precursor for 16-DPA synthesis.

Solasodine extraction from dried fruits with 70% 2- propanol gave crude solasodine glycosides as light brown powder, as compared to the product from leaves which had green powder characteristics. The crude glycosides obtained from the extraction were contaminated with coloured impurities since both coloured impurities and glycosides were soluble in alcohol. When the crude glycosides were repeatedly washed with water, better appearances were observed. This is due to the solubility of almost all coloured impurities in water. The crude glycoside yields were 2.03 and 2.17% w/w of the dry fruits and leaves, respectively. For hydrolysis of the crude glycosides, it was found that the proportion by weight of crude glycosides, 2-propanol and hydrochloric acid played a role to complete the acid hydrolysis reaction and give less side product reaction. The result of chromatograms indicated that hydrolysis of glycosides yielded the corresponding aglycone with practically less dehydrogenation product (solasodiene). After hydrolysis, the all-remaining impurities in crude solasodine were removed by filtration and crystallization. The average yield of white crystalline pure solasodine powder was 0.34 and 0.44% of dry fruits and leaves, respectively. The maximum yield of solasodine in dry fruits and leaves were 0.42 and 0.70%, respectively. The purity of solasodine was more than 90% (m.p. 198-200°C). This product has sufficient purity to be use as the starting material to convert to 16-DPA. All spectra (IR, MS and NMR) of the product were identical with that of the authentic sample. This indicated that solasodine can be isolated from both fruits and leaves of *S. laciniatum* by this method. It required no chromatographic procedure to eliminate the remaining impurities and colour materials. This method is simple, efficient and less expensive. It can also be applicable for the production of solasodine in large scale, since it will be more convenient to harvest both fruits and leaves or the whole plant at the same time in the isolation process.

### Example 2: Step1/Route B

### Synthesis of 16-DPA (16-dehydropregnenolone acetate, 3β-acetoxy-pregna-5,16-dien-20-one) from Solasodine Extracted from S. laciniatum, Ait Cultivated in Thailand

### 1. PCRNC method

A solution of solasodine (1 g) in acetic anhydride (5 ml), pyridine (7 ml) and p-toluenesulfonic acid (0.0250 g) was refluxed (160 °C) for 6 hrs. The reaction was cooled to room temperature and acetic acid (10 ml) and water (4 ml) were added and refluxed (160 °C) for 4 hrs. The mixture was cooled to 15 °C and a solution of chromic anhydride (0.75 g) in 80 % aqueous acetic acid (3 ml) was added dropwise with stirring over a period of 20 minutes. After the addition of the oxidant, the content was stirred for 4 hrs at room temperature and sodium sulphite (0.08 g) in water was added to decompose the excess oxidising agent. The mixture was allowed to stand overnight and acetic anhydride (5 ml) was added and refluxed (160 °C) for 6 hrs. The reaction was cooled to room temperature. The cool water was added to the precipitate of 16-DPA. The product was filtered, washed with water and dried, and recrystallised from methanol. It gave yellow powder. The product can also be purified by column chromatography on alumina neutral with n-hexane and ethyl acetate (9:1). It gave colourless needles of 16-DPA.

* % yield of 16-DPA = 44.6 from starting 10 g of solasodine.

Note: A phase transfer catalyst (PTC) may also be applied to increase the yield of 16-DPA, see below.

### 2. Alternative methods to synthesize 16-DPA from solasodine extracted from S. laciniatum, Ait cultivated in Thailand

### 2.1 Alternative method 1 (from J. Rodriguez, et al., J. Chem. Tech. Biotechnol, Vol. 29, 1979, 525-530)

### Acetylation and isomerisation of the diacetyl derivative.

A solution of solasodine (2 g, 80 %) in acetic acid (12 ml) was added to a solution of *p*-toluene-sulphonic acid (140 mg) in acetic acid (1.2 ml) and acetic anhydride (2.2 ml). The mixture was stirred at room temperature for 1 h and then heated under reflux for 6 h. The solution was then cooled to 15-18°C and after dilution with acetic acid (14 ml). The yields of 16-DPA, based on the theoretical conversion of solasodine, ranged from 15 of 20 %.

A mixture of solasodine (2 g, 80 %), acetic anhydride (1.8 ml) and pyridine (40 ml) was heated under reflux for 1 h. Then a solution of pyridine hydrochloride (3 g) in pyridine (50 ml) was added and refluxed for a further 2 h. The solvents were distilled off under vacuum and the residue was dissolved in acetic acid (60 ml). The following steps were performed as described below.

The yields of 16-DPA obtained varied between 20-30 %.

Solasodine (2 g, 80%) was dissolved in pyridine (40 ml) and heated under reflux. Acetic anhydride (2.3 ml) was added slowly and the reflux maintained for a further 1.5 h. The pyridine and excess of acetic anhydride were distilled off under vacuum. The residue obtained was treated with acetic acid (20 ml) and heated under reflux for 15 min. With this modification, the yields of 16-DPA were improved up to 48-53 %, based on the theoretical value obtained from pure solasodine.

### Oxidation and hydrolysis

The solution obtained after isomerisation, in acetic acid, was cooled to 15-20 °C and solution of sodium dichromate in acetic acid (11 %, 10 ml) was added, keeping the temperature below 40 °C. The mixture was stirred at room temperature for 1 h, then anhydrous sodium sulphite (0.52 g) was added to destroy the excess of oxidant, and the mixture heated for a further 2 h.

### Isolation and purification of 16-DPA

After attempting the isolation and purification of 16-DPA by various methods (crystallisation or extraction with benzene and chloroform), the following technique was finally adopted : the solution containing the 16-DPA was distilled under vacuum avoiding the complete drying of the residue. The rest of the acetic acid was eliminated by addition of benzene and distillation of the azeotrope formed. The residue was dissolved in a mixture of water-ether (1 :1, 100 ml). The ethereal layer was separated, the aqueous solution was extracted thoroughly with ether and the solvent was removed under vacuum.

A brown-yellow residue (3.3 g) was obtained, which was purified by crystallisation from methanol/water (4:1), or by chromatography over alumina with benzene as eluent. A very pure product was obtained (0.69 g) and characterised by UV, IR and NMR.

### 2.2 Alternative method 2 (modifjed from A.Sree, Y.R. Rao & S N Maha Patra, Research and Industry, Vol. 27 December 1982, pp 326-328)

### Acetylation and isomerisation of solasodine

*Pseudosolasodine acetate:* A solution of solasodine (100 g) in acetic anhydride (105 ml) and an amine solvent (650 ml) is distilled free of acetic acid and anhydride. The reaction mixture was cooled and water was added to decompose excess acetic anhydride. The crude product of *O,N*-diacetate was taken in 600 ml of acetic acid and refluxed for 1 hr to give the pseudosolasodine diacetate.

### Oxidation and hydrolysis

*16-dehydropregnenolone* acetate: The reaction mixture containing pseudosolasodine diacetate was cooled to 15 °C and a solution of chromic anhydride (45 g) in 200 ml 80 % aqueous acetic acid was added dropwise with stirring over a period of 20 minutes. After the addition of the oxidant, the contents were stirred for I hr at room temperature and sodium bisulphite (5 g) in water was added to decompose excess oxidising agent. The reaction mixture was refluxed for 3 hr, solvent was distilled off and water was added to precipitate 16-DPA. The product was filtered, washed and dried. It was thrice extracted with a hydrocarbon solvent. The extracts were combined and the solvent distilled off to get a colourless solid (44.5 g), m.p. 169-72°C.

### 16-Dehydropregnenolone Acetate:

A mixture of pyridine (40 ml, 0.05 mol) and NH₄Cl (26.0 g, 0.50 mol) is added at once to a stirred suspension of solasodine (210 g). The mixture is heated to 125-135 °C and kept at that temperature until TLC indicates the reaction to be complete (usually 8-9 h). Acetic acid (400 ml), 1. 2-dichloroethane (400 ml), and water (54 ml) are then added, and the mixture is cooled to 0 °C. A solution of CrO₃ (88.2 g, 0.882 mol) in water (124 ml) and acetic acid (42 ml) precooled to 0 °C is added dropwise, while the temperature is kept at 7 -10 °C. When 90 % (200 ml) of the solution has been added, cooling is discontinued. After the remaining portion has been added, the mixture is stirred for 1 h at room temperature. A solution of NaCl (100 g) in water (1.5 I) and methanol ( 16 ml) is then introduced and the stirring is continued for an additional hour. The keto ester is extracted with 1,2-dichloroethane (1 x 450 ml and 3 x 60 ml), and the combined extracts are washed with water (2 x 500 ml) to remove the residual chromium salts. Solid NaOAc x 3 H₂O (70 g, 0.51 mol) is added to the organic phase and the solvent is distilled off azeotropically to remove the water (4-6 h). The cooled residue is carefully treated with water (2 I) to produce a solid product, which is collected by filtration. The product is washed thoroughly with water until the filtrate is completely colourless. (To achieve complete removal of the residual chromium salts it may require a similar washing after 1 d). Crystallisation from methanol (400 ml) affords pure 16-dehydropregnenolone acetate (4); yield : 117.1-123.8 g (65.0-69.0 %); mp 167-172 °C.

### 2.3 Alternative method 3 (modified from US. Patent 5,808,117 , Chowdhury I et al., September 1998)

(a) Acetolysis of Solasodine to Pseudosolasodine diacetate 50 g of solasodine was charged with 40 ml of acetic anhydride and to it was added 150 ml of xylene in a pressure reactor vessel and heating was started while stirring till the temperature 200 °C and corresponding pressure of 5 kg/cm² are reached. The reaction was carried for a period of 10 hours after attainment of desired temperature (190 to 200 °C). Heating was stopped and was allowed to be cooled under stirring for a period of one hour and the product was discharged at a temperature less than 100 °C through the discharge tube. TLC (thin layer chromatography) analysis of the sample was carried out which showed only one major spot on TLC plate.
   Solvent removal was done in a rotary vacuum evaporator under reduced pressure (about 50 mbar). The recovered solvent was kept for recycle. After the removal of the last traces of the solvent, solid material was obtained which was confimed to be pseudosolasodine.
(b) Oxidation of pseudosolasodine to O,N-diacetate
   (i) Preparation of the oxidant solution. 25 g of chromium trioxide (CrO₃) was dissolved in 25 ml of water and 10 ml of glacial acetic acid to get a clean solution which was precooled to 0°C - 5°C.
   (ii) Addition of oxidant solution Pseudosolasodine diacetate obtained as above was dissolved in 100 ml of dichloroethane and 100 ml of glacial acetic acid and 25 ml of water. The mixture was cooled to 0 -5 °C and the oxidant solution prepared as above was added to it dropwise keeping the temperature of the reaction mixture below 5 °C till the addition was over. After the addition of the oxidant solution was complete, cooling was discontinued and the temperature of the reaction medium was allowed to rise up to 15 °C and also allowed to be stirred at that temperature for a period 25 minutes. When thin layer chromatography indicated the completion of the reaction, a solution of 5 g of sodium chloride in water (200 ml) and methanol (10 ml) were added and the stirring was continued for another 20 minutes.
   (iii) Extraction of *O,N*-diacetate
      The keto ester *O,N*-diacetate was extracted from the reaction mixture with 1, 2-dichloroethane (4 times, 200 ml). The organic layer after separation was subjected to distillation under reduced pressure to recover the solvent. A gummy residue of O, N- diacetate was obtained which was purified by column chromatography using petroleum ether and ethyl acetate.
(c) Hydrolysis and degradation of *O,N*-diacetate to 16-DPA. *O,N*-diacetate obtained above was allowed to reflux in 200 ml of glacial acetic acid for a period of 5 hr. The reaction was monitored on TLC. After completion of the reaction, acetic acid was recovered by distilling under reduced pressure (50 mbar). The cooled residue was then treated with cold water (1 I) to remove maximum amount of chromium salts present and as a result solid 16-DPA separated out which was collected by filtration.

The residue was thoroughly washed with cold water 5 times. Finally, the solid residue was subjected to exhaustive extraction with 1.5 I of petroleum ether (b.p. 60 - 80 °C) when a yellow solution of 16-DPA was obtained leaving a black residue.

The yellow solution on distillation gave crude yellow 16-Dehydropregnenolone acetate which was recrystallized from ethanol to get creamy white crystals. The yield of 16-DPA was found to be 60 %.

### 2.4. Alternative method 4: Synthesis of 16-DPA from solasodine by Phase transfer catalysis (PTC)

### 2.4.1 Acetylation and isomerisation

A mixture of solasodine (1 g), pyridine (10 ml) and acetic anhydride (5 ml) was refluxed for 2 h and cooled. Then, the mixture was poured on ice plus aqueous ammonia solution. The precipitate was filtered and dried. The product was refluxed in glacial acetic acid (10 ml) for 1 h. Glacial acetic acid was distilled off under vacuum giving a yellow residue.

### 2.4.2 Oxidation and hydrolysis

### 2.4.2.1 Conventional technique

For hydrogen peroxide as an oxidizing agent, the residue from 2.4.1 was refluxed with glacial acetic acid (10 ml) and hydrogen peroxide (30 mol % excess) at 70°C for 5 h, and then water was added to destroy excess oxidizing agent. The reaction mixture was extracted with ethyl acetate and the solvent was evaporated. The residue was refluxed in glacial acetic acid (10 ml). For potassium permanganate, potassium dichromate and chromic trioxide as oxidizing agents, the solution obtained after isomerisation in glacial acetic acid was cooled to 0 to 15°C and a solution of potassium permanganate, potassium dichromate or chromic trioxide (30 mol % excess) in glacial acetic acid (10 ml) was added dropwise with stirring for 3 h. Then, methanol was added to destroy the excess oxidizing agent. The reaction mixture was refluxed for 2 h and the solvent was distilled off under vacuum.

### 2.4.2.2 Phase-transfer catalysis technique

The residue from 2.4.1 was dissolved in methylene chloride (10 ml). The oxidizing agent (30 mol % excess) and phase-transfer catalyst (tetraethylammonium iodide or tetrabutylammoium hydrogen sulfate; 10 mol % of oxidizing agent) were dissolved in water (10 ml) and added to the reaction mixture dropwise. The mixture was vigorously stirred while maintaining the pH at 0 to 1 by adding sulfuric acid. The bath temperature was maintained at 0 to 15°C for 3 h. The organic solvent phase was separated from the system and washed with water several times. Then, the solvent was removed under vacuum until gummy material was obtained. The product was refluxed in glacial acetic acid ( 10 ml) for 2 h and distilled off under vacuum.

### 2.4.3 Purification

The gummy material of the product was packed on a column of silica gel 60 (Merck, Germany) and eluted with petroleum ether and ethyl acetate with increasing polarity. The eluent gave pure 16-DPA. Recrystallisation of 16-DPA from methanol gave a rod-like crystal.

### 2.4.4. Analysis of solasodine and 16-DPA

### 2.4.4.1 Qualitative analysis

The isolated solasodine was preliminary detected by thin-layer chromatography (TLC) by comparison with an authentic sample using silica gel 60 GF₂₅₄ aluminium plate (Merck, Germany) developed with a mobile phase consisting of chloroform / methanol (9:1). The spots were visualized by spraying with 30% v/v sulfuric acid solution on TLC plate before heating on a hot plate. 16-DPA was detected in the same manner but developed with mobile phase consisting of ethyl acetate / petroleum ether (2:8). The spots were detected under UV lamp at 254 nm.

### 2.4.4.2 Quantitative analysis

Solasodine was analyzed by high performance liquid chromatography (HPLC, Thermo Separation Products Inc., Califomia, USA) at 205 nm using a Lichrosorb C-18 column (250x4.6 mm i.d.; 10 µm particle diameter, HPLC Technology, UK) and 0.01 M Tris-HCI buffer/ acetonitrile (20:80 by volume) as the mobile phase at a flow rate of 2.00 ml/min with injection volume of 20 µl. 16-DPA was analyzed by HPLC at 254 nm using a Hypersil C-18 column (250x4.6 mm i.d.; 10 µm particle diameter; 250°A average pore size, Hichrom, UK) and 100% methanol was used as the mobile phase at a flow rate of 1.00 ml/min with injection volume of 5 µl.

### 2.4.4.3 Identification

Solasodine and 16-DPA were determined by melting point (m.p.) measurement (Stuart Scientific, UK), IR (Jasco *FTIIR-5000,* Japan), GC-MS (Varian Satum 2100, UK) and NMR (Hitachi R-1500, Japan) comparing to the authentic standards.

### 2.5. Results and Discussion of alternative method 4

KMnO₄, CrO₃, Na₂Cr₂O₇, or K₂Cr₂O₇ may be used as oxidising agents. Tetraethylammonium iodide or tetrabutylammoium hydrogen sulfate may be used as phase transfer catalysts. Best yield of 16-DPA with respect to solasodine was obtained with tetrabutylammonium hydrogen sulfate and K₂Cr₂O₇ (37.0 %).

Solasodine was first converted to pseudosolasodine diacetate as in two consecutive steps: i.e., acetylation of solasodine to give *O,N*-diacetylsolasodine and isomerisation of diacetate to give pseudosolasodine diacetate. The resulting residue was oxidized by various oxidizing agents under PTC and conventional methods. Further hydrolysis of the oxidative product produced 16-DPA. Finally, the product was separated by column chromatography. The highest yield (37.0%, 93% purity , m.p. 169-172°C) based on the theoretical of solasodine by continuous operation without purification of intermediates was found in PTC system using tetrabutylammonium hydrogen sulfate and potassium dichromate. The product characterized by m.p., IR, MS and NMR were identical to the authentic sample. Potassium dichromate under tetrabutylammonium hydrogen sulfate as phase-transfer catalyst gave better yield than tetraethylammonium iodide and the conventional technique.

In conventional technique, the yield of the product obtained by potassium dichromate was higher than from potassium permanganate. However, even being a strong oxidizing agent, chromium trioxide can not be used for PTC since it is not ionized. In case of hydrogen peroxide, no oxidation product can be observed because. of its low oxidizing potency.

In our novel method of 16-DPA production by oxidation of pseudosolasodine diacetate using potassium dichromate as an oxidizing agent under PTC, not only the product can be separated easily from the system, but also the excess of the active dichromate salts in the aqueous phase can be reused as well. In addition, less toxic and less amount of chromium were used. Hence, low cost and low waste production, which will be advantageous for commercial production scale of 16-DPA can be anticipated.

### Example 3: Step 2/Route B

### Synthesis of 16, 17α-epoxypregnenolone trom 16-dehydrogregnenolone acetate (16-DPA) (Percy L. Julian, et at., J. Am. Chem. Soc., Vol. 72, 1950, pp. 5145-5147)

Three grams of 5,16-pregnadiene-3β-ol-20-one acetate was dissolved in 200 ml of methanol. This solution was treated, after chilling to 15°C, with 6 ml of 4 N sodium hydroxide solution and then immediately with 12 ml of 30 % hydrogen peroxide solution. The mixture was then stored in the refrigerator at 5°C for twenty-three hours. The methanol solution was poured into 800 ml of water and the resulting white solid separated. The solid, which was washed well with water and dried, weighed 2.63 g (95 %) and melted at 180-186°C. Recrystallization of a sample from methanol gave colourless needles melting at 187 -190°C.

* % yield = 100.43 from 8 g of starting materials.

### Example 4: Step 3/Route B

### Synthesis of 16-Bromo-17α-hydroxy-pregnenolone from 16, 17α-epoxypregnenolone (H. J. Ringold, et al., J. Am. Chem. Soc., Vol. 78, 1956, pp. 816-819)

A solution of hydrogen bromide in glacial acetic acid (215 ml, 32 % w/v) was added during 10 minutes to a stirred suspension of 107 g of 16α, 17α-oxido-Δ⁵-pregnen- 3β-ol-20-one in 1.1 I of glacial acetic acid, the internal temperature being kept at 18°C by ice- cooling. A homogeneous solution resulted a few minutes after the end of the addition, followed shortly thereafter by partial crystallisation of the bromohydrin. After a reaction time of 25 minutes at 18°C, the mixture was poured into 7 I of water, the crude bromohydrin was collected, thoroughly washed with water and air-dried for 24 hours at room temperature.

* % yield = 88.57 (42.13 + 46.44) from 5 g of starting materials.

### Example 5: Step 4 / Route B

### Synthesis of 17α-hydroxypregnenolone from 16-Bromo-17α-hydroxypregnenolone (Bromohydrin) (H.J. Ringold, et. al., J. Am. Chem. Soc., Vol. 78, 1956, pp. 816-819)

The total crude bromohydrin was suspended in 5 I of 96% ethanol together with 400 g of Raney-Nickel. The suspension was heated to boiling, the catalyst was removed and washed thoroughly with boiling ethanol. The combined filtrates were concentrated to 500 ml, cooled in ice and the resulting precipitate was collected. This procedure produced 91.0 g of Δ⁵-pregnene-3β, 17α-diol-20-one-3-acetate.

* % yield = 84.19 (41.57 + 42.62) from 5 g of starting materials.

### Example 6: Step 5/Route B

### Synthesis of 5-pregnene-3β, 17α-diol-20-one-3-formate (formate) from 17α-hydroxypregnenolone (H. J. Ringold, et al, J. Am. Chem. Soc., Vol. 78, 1956, pp. 816-819)

A suspension of 90 g of Δ⁵-pregnene-3β, 17α-diol-20-one in 2.3 I of 85% formic acid was stirred for 2 hours at 70°C. A homogeneous solution did not result at any time but a change of crystal form was noted. The mixture was cooled in ice and the formate was collected. It weighed 81.0 g (83 %) and showed a m.p. of 203-207°C. A sample was crystallised from acetone and then exhibited a m.p. of 207-209°C.

* % yield = 98.83 from 10 g of starting materials.

### Example 7: Step 6/Route B

### Synthesis of 5-pregnene-3β, 17α-diol-20-one 3-formate 17-acetate (diester) from 5-pregnene-3β, 17a-diol-20-one 3-formate (formate) (H. J. Ringold, et al, J. Am. Chem. Soc., Vol. 78, 1956, pp. 816-819)

A mixture of 100 g of the formate, 2.5 g of p-toluenesulfonic acid hydrate and 400 ml of acetic anhydride was heated with stirring at 80° for 30 minutes, allowed to stand at room temperature for 2 hours and finally overnight at 0°C. The diester was collected, washed first with a little cold acetic anhydride and then with hot water. The dried product weighed 99.5 g (89 %) and showed a m.p. of 192-196°C. The analytical sample was obtained through crystallisation from acetone and exhibited m.p. 198-200°C.

* % yield = 94.78 from 10 g of starting materials.

### Example 8: Step 7/Route B

### Synthesis of 17α-acetoxyprogesterone from 5-pregnene-3β, 17α-diol-20-one 3-formate 17-acetate (diester) (H. J. Ringold, et al., J. Am. Chem. Soc., Vol. 78, 1956, pp. 816-819.)

The diester (51 g, m. p. 192-196°C) was dissolved in 1.4 I of commercial xylene and 510 ml of cyclohexanone and the solution was distilled (250 ml of distillate collected) to remove moisture. Aluminum isopropoxide (51 g) dissolved in 210 ml of xylene was added during 5 minutes to the slowly distilling solution and distillation was then continued for an additional 45 minutes (further 210 ml of distillate collected). The mixture was cooled rapidly, a mixture of ice and water was added and the solvents were removed through steam distillation. The resulting solid was collected by filtration and dried. Extraction with hot acetone, followed by crystallisation from this solvent, furnished 40.7 g (86 %) of 17α-acetoxy-progesterone with m.p. 240-243°C. A further purified sample showed a m.p. of 243-247°C.

* % yield = 78.95 from 2 9 of starting materials.

### Example 9: Step 8/Route B

### Synthesis of 17α-Acetoxy-4,6-pregnadiene-3,20-dione from 17α-acetoxyprogesterone (Giichi Goto, et al., Chem. Pharm. Bull., Vol. 26 (6), 1978, pp. 1718-1728)

To a solution of 17α-acetoxyprogesterone (5.0 g) in t-BuOH (50 ml) was added chloranil (4.0 g) and the reaction mixture was heated to 80° for 5 hr. After cooling, the reaction mixture was poured into 10 % Na₂CO₃ solution and the products were extracted with CH₂Cl₂. The extracts were washed with water dried over Na₂SO₄ and the solvent was evaporated to afford crude crystals. Recrystallisation from acetone gave (4.6 g) colourless needles.

* % yield = 98.53 from 4 g of starting materials.

### Example 10: Step 9 / Route B

### Synthesis of 17α-acetoxy-6, 7 α-oxido- 4-pregnene-3, 20 -dione from 17 α-acetoxy-4,6-pregnodiene-3, 20-dione (Step 9-2/route B)

### 1. PCRNC method

1.0 g of 17α-acetoxy-4,6-pregnadien-3, 20-dione were dissolved in 30 ml of dichoromethane and added with 0.9 g m-chloroperbenzoic acid. The solution was allowed to stand at room temperature for 24 hrs. The reaction mixture was poured in to 5 % sodium carbonate solution and the product was extracted with dichloromethane. The extracts were washed with water, dried over anhydrous sodium sulphate. The solvent was evaporated to 17a-acetoxy-6, 7 -oxido-4-pregnene-3,20-dione (colourless oil). The residue was subjected to chromatography over silica gel, using a petroleum ether and ethyl acetate mixture (3:2), and gave a colourless needle.

* % yield = 95.02 from 1 g of starting materials.

### 2. Alternative methods for the synthesis of 17α-acetoxy-6, 7 α-oxido-4- pregnene-3, 20 -dione from 17 α-acetoxy-4,6-pregnadiene-3, 20-dione

### 2.1 Alternative method 1 (US Patent 3,234,093, Rudoff Wiechert, et al., 1966)

2.34 g of 1, 2 α-methylene-Δ^{4,6}- pregnadiene-17α-ol-3, 20-dione-17-acetate are dissolved in 18.25 ml of ethylene chloride which contains 844 mg of perbenzoic acid. The solution is stored for 16 hours at +5 °C and 7 hours at room temperature. It is then diluted with methylene chloride and with aqueous ferrous sulfate solution, sodium bicarbonate solution and washed into neutral with water.

### 2.2 Alternative method 2 (Giichi Goto, et, al., Chem, Pharm. Bull, Vol. 26 (6), 1978, pp. 1718-1728.)

To a solution of 17 *β-acetoxy-1,2α*-methylene-6α,7α-epoxy-16β-isopropylandrost-4en-3-one (1.5 g) in CH₂Cl₂ (30 ml) was added m-chloroperbenzoic acid (0.9 g) and the solution was allowed to stand at room temperature for 24 hr. The reaction mixture was poured into 5 % Na₂CO₃ solution and the product was extracted with CH₂Cl₂. The extracts were washed with water, dried over Na₂SO₄ and the solvent was evaporated.

### 2.3 Alternative method 3 (U.S. Patent 3485852, Howard J. Ringold, 1969)

A solution of 4 g of 19-nor-Δ^{4,6}- pregnadien-17α-ol-3,20-dione 17-acetate in 100 ml of chloroform was cooled to 0 °C and then admixed with 4 molar equivalents of monoperphthalic acid dissolved in diethyl ether. The resulting reaction mixture was held at room temperature for 20 hours, after which it was diluted with water. Next, the organic layer was separated, washed with an aqueous sodium bicarbonate solution and then with water until neutral, then dried over anhydrous sodium sulfate and finally evaporated to dryness. Recrystallisation of the dry residue from acetone/hexane gave 6α, 7α-oxido-19- nor-Δ⁴-pregnen-17α-ol-3,20-dione-17-acetate. This procedure was then repeated in every detail but one, namely, Δ^{4,6}-pregnadien-17α-ol-3,20-dione-17-acetate was used as the starting steroid, thus giving 6α,7α-oxido-Δ⁴-pregnen-17α-ol-3,20-dione-17-acetate.

### 2.4 Alternative method 4 (German patent 1158966, Rudolf Wiechert, et al., 1963.)

2.34 g of 1,2α-methylen-Δ^{4,6}-pregnadien-17α-ol-3,20-dione-17-acetate are dissolved in 18.25 ml ethylenechloride containing 844 mg perbenzoic acid. The solution is kept at +5 °C for 16 hours and 7 hours at room temperature. Afterwards, it is diluted with methylene chloride and washed with aqueous iron (II) sulfate solution, sodium hydrogen carbonate solution, and water until neutral. After drying, the organic phase is reduced over sodium sulfate till dryness. 1.62 g of the crude 1,2α-methylene-6,7α-oxido-Δ⁴-pregnen-17α-ol-3,20-dione-17-acetate thus obtained are dissolved in 109 ml glacial acetic acid. This solution is saturated with hydrogen chloride gas and stored at room temperature for 20 hours. It is then diluted with methylene chloride and washed neutral with water. After drying, the organic phase is reduced to dryness over sodium sulfate. The crude 6-chloro-1α-chloromethyl-Δ^{4,6}-pregnadien-17α-ol-3,20-dione-17-acetate thus obtained is added to 20 ml collidine and heated under nitrogen to boiling for 20 minutes. After dilution with diethylether, the reaction mixture is washed neutral with 4 N HCI and water.

The residue after drying above sodium sulfate and reduction to dryness is chromatographed over silica gel. 900 mg 6-chloro-1,2α-methylene-Δ^{4,6}-pregnadien-17α-ol-3,20-dione-17-acetate are eluted with a mixture of benzene and acetic acid ester (19:1) which , after recrystallisation from isopropyl ether, has a m.p. of 200-201 °C. UV = ε₂₈₁ = 17820 (methanol).

### Example 11: Step 10/Route B

### Synthesis of chlormadinone acetate from 6,7a-oxido-4-pregnene (step 10-2/route B)

### 1. PCRNC method

3.3640 g of 17α-acetoxy-6,7α-oxido-4-pregnene-3,20-dione were dissolved in 80 ml glacial acetic acid. A slow current of anhydrous hydrogen chloride gas was passed through the suspension for 4 hrs, stored for 20 hrs, and then poured into ice water. The precipitate is formed by filtration, gave pale-yellow powder of chlormadinone acetate.

* % yield of chloromadinone = 91.18 from starting 6,7α-oxido-4-pregnene

### 2. Alternative methods to prepare chlormadinone acetate from 6,7α-oxido-4-pregnene

### 2.1 Alternative method 1 (US. Patent 3485852, Howard J. Ringold, 1969)

One gram of 6α,7α-oxido-19-nor-Δ⁴-pregnen-17α-ol-3,20-dione-17-acetate was suspended in 35 ml of glacial acetic acid. Next, a slow current of anhydrous hydrogen chloride gas was passed through the suspension, and in 10 minutes all the solid matter present had dissolved. The passage of the gas was continued for a total of 5 hours, then the solution was concentrated to about one-third of its initial volume by distillation under reduced pressure at 35°C, and then poured into ice water. The precipitate formed thereby was collected by filtration, washed with water until neutral, and then dried. Recrystallization from methylene chloride/hexane gave 6-chloro-19- nor-Δ^{4,6}-pregnadien-17α-ol-3,20-dione-17-acetate.

By repeating this procedure in every detail with one exception, namely, replacing the anhydrous hydrogen chloride gas with anhydrous hydrogen bromide gas, the corresponding 6-bromo-19-nor-Δ^{4,6}-pregnadien-17α-ol-3,20-dione-17-acetate was obtained.

Similarly, by replacing the starting steroid with 6α,7α-oxido-19-nor-A4-pregnen-17α-ol-3,20-dione-17-acetate and the anhydrous hydrogen chloride gas with anhydrous hydrogen bromide gas, keeping all other factors the same, 6-bromo-Δ^{4,6}-pregnadien-17α-ol-3,20-dione-17-acetate was obtained.

### 2.2 Alternative method 2 (US. Patent 3234093, Rudolf Wiechert, et al, 1966)

1.62 g of crude 1,2α-methylene-6α,7α-oxido-Δ⁴-pregnen-17α-ol-3,20-dione-17-acetate are dissolved in 109 ml of glacial acetic acid. This solution is then saturated at room temperature with hydrogen chloride gas and stored for 20 hours. It is then diluted with methylene chloride and washed with water until neutral.

The organic phase is dried over sodium sulfate and then concentrated to dryness.

### 2.3 Alternative method 3 (Giichi Goto, et , al., Chem. Pharm. Bull., Vol. 26(6), 1978, pp. 1718-1728)

A solution of 17β-acetoxy-1,2α-methylene-6α,7α-epoxy-16β-isopropylandrosta-4,6- dien-3-one (2.0 g) in glacial acetic acid (20 ml) was saturated with anhydrous hydrogen chloride. After standing at room temperature for 20 hr, the reaction mixture was poured into ice-water and the product was extracted with CH₂Cl₂. The extract were worked up in the usual manner to give 17β-acetoxy-1α-chloro-methyl-6-chloro-16β-isopropylandrosta-4,6-diene-3-one (1.74 g) was dissolved in γ-collidine (15 ml) and refluxed under N₂ for 30 min. After cooling, the reaction mixture was extracted with CH₂Cl₂. The extracts were washed with 5 % HCI solution, water, and dried over Na₂SO₄. Evaporation of the solvent gave crude crystals. Recrystallisation from ether gave 1.4 g as colourless needles.

### Example 12: Step 11 / Route B

### Synthesis of delmadinone acetate from chlormadinone acetate (step 11/Route B)

### 1. PCRNC Method

### 1.1 Chemical synthesis

1.0 g chlormadinone acetate were dissolved in dioxane (40 ml) and an addition of 2,3- dichloro-5,6-dicyano-1,4-benzoquinone (DDQ)( 0.636 g). The reaction mixture was refluxed for 6 hrs (110 °C). After cooling to room temperature, the solution passed through short column of neutral alumina and the products were eluted with ethyl acetate. The eluent were evaporated to dryness, gave pale-yellow oil of delmadinone acetate.

* % yield of delmadinone acetate = 81.43 from 1 g of chlormadinone

### 1.2 Biotechnological synthesis

Cultivation of bacterial cells. Two strains of standard bacteria, *A. simplex* (ATCC 6946) and *B. sphaericus* (ATCC 13805), which were kept in mineral oil layered agar slants, were subcultured in TSA (Tryptic Soy Agar, Difco, USA) for 48 h. Each strain was grown in 100 ml first-stage cultivation medium containing (g/l) in 50 mmol Tris-HCI buffer pH 7.8: yeast extract, 5; ammonium sulfate, 3; and magnesium chloride, 0.1; in 250 ml shaken flasks at 25 ± 2°C, 200 rpm for 48 h. The bacterial cell concentrations were primarily determined by plate count method.

Biotransformations. Each portion of the bacterial cell suspension was added to the second-stage cultivation medium adjusted to 50.0 ml, which was the same as first- stage cultivation medium. The cultures were then grown in 250-ml shaken flasks at 25 ± 2°C, 200 rpm for 48 h. Various chemicals as described below were added to the mediums (the previous optimal results of each effect for each bacterial cells have been used in further studies). The conditions were maintained as described above and all cultivation reactions (0.5 ml each) were collected for HPLC analysis at 0, 4, 24, 48, 72 and 96 h.

To optimise the effects of exogenous electron carrier, chlormadinone acetate and various concentrations of menadione (0-1.2 mmol/l) dissolved in dirnethylformamide (DMF) (5%) were added to the media.

To optimise the effects of substrate and organic solvent, menadione and various concentrations of chlormadinone acetate (0.12-0.50 mmol/l) and DMF (5 or 10 %) were added to the media.

To optimise the effects of steroid inducers, hydrocortisone (0-0.55 mmol/l) as a 2% ethanol solution was added to the media. After 24 hour cultivation in the presence of hydrocortisone, menadione and chlormadinone acetate in DMF were added.

To optimise the effects of surfactant, menadione, chlormadinone acetate and various concentrations of Tween 80 (0-1.00% w/v) in DMF were added to the media after 24 h cultivation in the presence of hydrocortisone.

To optimise the effects of D(+)-glucose, various contents of D(+)-glucose (0-10.0 g/l) were contained in the second- stage cultivation medium. Menadione, chlormadinone acetate and Tween 80 in DMF were added after 24 h cultivation in the presence of hydrocortisone.

HPLC analysis. The samples (0.5 ml) taken from the cultivation mixture were extracted with 2.0 ml of chloroform by vortex mixing for 2 min. A portion (0.5 ml) of chloroform phase was transferred to a sampling vial. Chlormadinone acetate and delmadinone acetate concentrations were determined by high performance liquid chromatography (HPLC, Thermo Separation Products Inc., USA) at 282 nm. Each sample (5 µl) was analyzed on a 250x4.6 mm Hypersil 5 µ ODS 250A column (Hichrom Inc., USA) employing methanol / water (70:30 by volume) as the mobile phase at a flow rate of 1.0 ml/min. Delmadinone acetate and chlormadinone acetate gave completely separations from other compounds with retention times at 9.3 and 11.3 min, respectively. The other detected compounds were hydrocortisone, prednisolone and menadione.

The reaction mixture without menadione had very low productivity in both A. *simplex* and *B. sphaericus*. All cultivations of *B. sphaericus*. In the presence of menadione ranging from 0.3 to 1.2 mmol/l gave delmadinone acetate yields 3-5 times higher than cultivations without exogenous electron carrier. But at higher concentration than 0.3 mM, menadione apparently did not increase the product yield.

The decrease of substrate concentrations from 0.50 to 0.12 mmol/l both in 5% and 10% DMF lead to a more complete conversion of the substrate for *B. sphaericus*, with the highest yield of about 15% at 0.12 mmol/l chlormadinone acetate in 5% DMF.

All conditions of *A. simplex* with the steroid inducer gave the maximum product at 4 h, which was more than without hydrocortisone. Hydrocortisone did not only induce the enzyme activity for the biotransformation of chlormadinone acetate but was also completely converted to prednisolone as well.

Tween 80 concentrations of 0.25 to 1.00 % (w/v) improved yield in A. simplex, with a preferred range from 0.5 to 1.00 %. The concentration of Tween 80 at 0.75% w/v gave the highest yield at 28.7% on *A. simplex.*

The chemical dehydrogenation of chlormadinone acetate by DDQ is commonly used with higher yield, but especially in the large-scale production, this process uses large amounts of the toxic reactant (DDQ) and organic solvent (dioxane) must directly effect to environment. Another problem is the production of hydroquinone from DDQ, which has to be disposed together with the product from the column chromatography. This further increases the cost. On the other hand, biotransformation of chlormadinone acetate to delmadinone acetate by using free cells of A. *simplex* or *B. sphaericus*. in aqueous media can be used as an advantageous alternative pathway for clean synthesis. The optimal conditions for the biotransformation of the starting 0.25 mmol/l chlormadinone acetate for *A. simplex* were 5% DMF, 0.41 mmol/l hydrocortisone, and 0.75% w/v Tween 80 and of the starting 0.12 mmol/l chlormadione acetate for *B. sphaericus'* were 0.6 mmol/l menadione, 5% DMF, 0.41 mmol/l hydrocortisone and 0.25 g/l D(+)-glucose. The overall effects in both strains gave delmadinone acetate production less than 40%.

The techniques of two-phase system and cell immobilization were also developed for the biotransformation of chlomadinone to delmadinone.

The application of liposomes to solubilise the substrates and products of the above experiment was also introduced to increase the biotransformation yields.

### 2. Alternative Methods

### 2.1 Alternative method 1 (Anthony Y. et al., Steroids, Vol. 62 p.221-225t 1997)

Dichlorodicyanobenzoquinone (100 mg, 3.3 equiv.) was added to a solution of chlormadinone acetate (63 mg, 1.45 x 10⁻⁴ mole) in dioxan (2 ml) and refluxed overnight while stirring. After cooling to room temperature the reaction mixture was filtered. Three drops of pyridine were added and evaporated to dryness. The residue was extracted with ethyl acetate and washed with saturated aqueous sodium bicarbonate, water dried (Na₂SO₄) and evaporated to give 50 mg (80 %).

### 2.2 Alternative method 2 ( Bernardo Beyer. et al. Steroids. Vol. 31, pp. 481-488, 1980)

A solution of chlormadinone acetate (1 g. 2.68 mmol) and 2,3-dichloro-5.6-dicyanobenzoquinone (0.92 g. 4.05 mmol) in dioxane (40 ml) was refluxed for 16 hr and then cooled. The precipitated hydroquinone is formed and the solvent from the filtrate was removed under reduced pressure. The resultant dark solid was chromatographed over activated magnesium silicate. Elution with benzene-CHCl₃ afforded a white solid 61 %)

### 2.3 Alternative method 3 (Giichi Goto. et al., Chem. Pharm. Bull. Vol. 26 (6). 1987, p.1718-1728)

To a solution of chlormadinone acetate (3.5 g) in dioxane (40 ml) was added DDQ (2.8 g) and the reaction mixture was refluxed for 5 hr. After cooling, the solution was treated in the same manner. Recrystallisation from ether gave delmadinone acetate (3.2 g) as colourless needles.

### Example 13: Step 12/Route B

### Synthesis of cyproterone acetate from delmadinone acetate

1.2 g of trimethyl sulfoxonium iodide (5.6 mmol) and 0.08 g of sodium hydride (3.3 mmol) were dissolved in 10 ml dimethylsulfoxide (DMSO) and stirred for 30 minutes at 5°C under nitrogen gas. Then, 0.5 g of delmadinone acetate (1.24 mmole) were added and stirred for 5 hours. The mixture was allowed to room temperature for 24 hours. The mixture as poured into 1 N HCI (with ice) solution, the precipitate filtered and washed with water. Recrystallisation from isopropylether gave white powder of cyproterone acetate.

* % yield = 51.51 from 0.5 g of starting materials.

## Claims

1. Process for the production of cyproterone acetate (M), comprising
(a) Converting 6,7α-oxido-4-pregnene-17α-ol-3,20-dione-17-acetate (J₂) into chlormadinone acetate (K₂) by a single step procedure;
(b) Introducing a double bond at position 1 of (K₂) to yield delmadinone acetate (L₂); and
(c) Introducing a methylene group bridging positions 1 and 2 of (L₂) to yield cyproterone acetate (M).

2. Process according to claim 1, wherein 6,7α-oxido-4-pregnene-17α-ol-3,20-dione-17-acetate (J₂) is produced by a process comprising
(d) Introducing a double bond at position 6 of 17α-acetoxyprogesterone (H) to convert it into 4,6-pregnadiene-17α-ol-3,20-dione-17-acetate (I₂);
(e) Converting the double bond at position 6 of (I₂) into an epoxy function to yield 6,7α-oxido-4-pregnene-17α-ol-3,20-dione-17-acetate (J₂).

3. Process according to claim 1 or 2, wherein step (a) is carried out by passing anhydrous hydrogen chloride gas through a reaction mixture containing 6,7α-oxido-4-pregnene-17α-ol-3,20-dione-17-acetate (J₂).

4. Process according to anyone of claims 1 to 3, wherein step (b) is carried out by the use of 2,3-dichloro-5,6-dicyano-benzoquinone (DDQ).

5. Process according to anyone of claims 1 to 4, wherein step (c) is carried out using trimethyl sulfoxonium iodide (TMSI) and an alkali hydride, preferably sodium hydride.

6. Process according to any one of claims 2 to 5, wherein step (d) is carried out using chloranil.

7. Process according to anyone of claims 2 to 6, wherein step (e) is carried out using m-perbenzoic acid or monoperoxyphthalic acid.

8. Process for the production of cyproterone acetate (M), comprising
(f) Introducing two double bonds at positions 1 and 6 of 17α-acetoxyprogesterone (H) to convert it into the 1,4,6-triene compound (I₁) by a single step procedure;
(g) Introducing an methylene group bridging positions 1 and 2 to yield the 1,2α-methylene compound (J₁);
(h) Introducing an oxido group bridging positions 6 and 7 of (J₁) to yield the 6,7α-epoxy compound (K₁);
(i) Transforming the epoxy group of (K₁) to the 6-chloro-7-hydroxy compound (L₁); and
(j) Converting (L₁) to cyproterone acetate (M).

9. Process according to claim 8, wherein DDQ is used in step (f).

10. Process according to claim 8 or 9, wherein TMSI and an alkali hydride are used in step (g).

11. Process according to any one of claims 8 to 10, wherein trifluoromethyl sulfonyl chloride and lithium chloride are used in step (i).

12. Process according to any one of claims 1 to 11, wherein 17α-acetoxyprogesterone (H) is produced by a process comprising converting solasodine (A) into 17α-acetoxyprogesterone (H) by means known in the art.

13. Process according to claim 12, wherein 16-dehydropregnenolone acetate (B) is converted into 16,17-epoxy-pregnenolone (C) by a single step procedure.

14. Process according to claim 13, wherein the conversion of (B) to (C) is achieved using hydrogen peroxide in alkaline solution.

15. Process for the production of cyproterone actetate (M), comprising reacting delmadinone acetate (L₂) with TMSI and an alkali hydride, preferably sodium hydride.

16. Process for the production of cyproterone actetate (M), comprising reacting compound (K₁) of formula 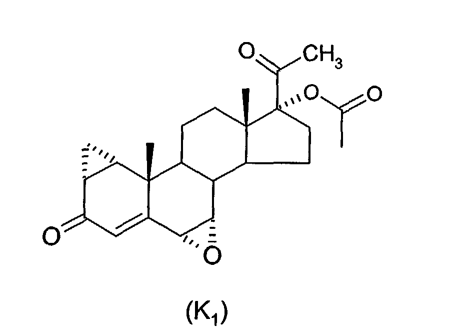 with lithium chloride/trifluoromethyisulfonylchioride or lithium chloride/N,N-dimethyl acetamide hydrochloride to yield compound (L₁) of formula 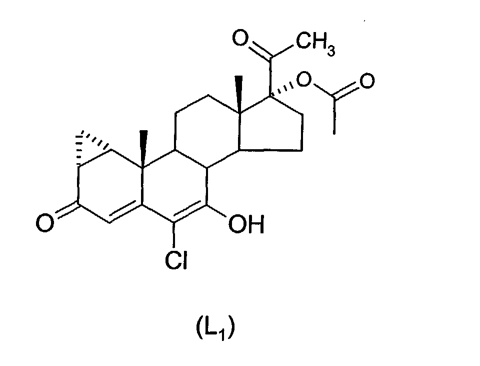 and reacting compound (L₁) with an aqueous acetate solution, preferably a solution of sodium acetate, to yield cyproterone acetate (M).

17. Process for the production of 16-dehydropregnenolone actetate (B), comprising
(k) Acetylating solasodine (A) to give *O, N*-diacetylsolasodine;
(I) Isomerising *O, N*-diacetylsolasodine to give pseudosolasodine diacetate;
(m)Oxidising the resulting pseudosolasodine diacetate in the presence of a phase transfer catalyst.

18. The process of claim 17, wherein the phase transfer catalyst is tetraethylammonium iodide or tetrabutylammonium hydrogen sulfate.

19. The process of claim 17 or 19, wherein the oxidising agent is potassium dichromate, sodium dichromate, potassium permanganate, or chromium trioxide.

20. Process of production of delmadinone acetate (L₂) comprising adding chlormadinone acetate (K₂) to a culture of a microorganism capable of converting (K₂) into (L₂), and isolating the resulting delmadinone actetate from the culture.

21. The process of claim 20, wherein the microorganism is *Arthrobacter simplex* or *Bacillus sphaericus.*

22. The process of claim 21, wherein is *Arthrobacter simplex* is ATCC 6946, or *Bacillus sphaericus* is ATCC 13805.
The process of any one of claims 20 to 22, wherein an electron carrier is added to the culture, preferably menadione (2-methyl-1,4-naphthoquinone).

23. The process of any one of claims 20 to 23, wherein a steroid is added to the culture, preferably hydrocortisone.

24. The process of any one of claims 20 to 24, wherein a surfactant is added to the culture, preferably polyoxyethylenesorbitan monooleate.
